(19) Europäisches Patentamt
European Patent Office
Office européen des brevets

(11) Veröffentlichungsnummer: **0 002 215**
**A1**

(12) # EUROPÄISCHE PATENTANMELDUNG

(21) Anmeldenummer: **78101396.6**

(22) Anmeldetag: **18.11.78**

(51) Int. Cl.$^2$: **C 07 C 177/00**

(30) Priorität: **03.12.77 DE 2753995**

(43) Veröffentlichungstag der Anmeldung:
**13.06.79 Patentblatt 79/12**

(84) Benannte Vertragsstaaten:
**DE**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Lieb, Folker, Dr.**
**Alfred-Kubin-Strasse 1**
**D-5090 Leverkusen(DE)**

(72) Erfinder: **Busse, Wolf-Dieter, Dr.**
**Pahlkestrasse 65**
**D-5600 Wuppertal 1(DE)**

(72) Erfinder: **Oediger, Hermann, Dr.**
**Roggendorfstrasse 51**
**D-5000 Köln 80(DE)**

(72) Erfinder: **Sitt, Rüdiger, Dr.**
**Claudiusweg 9**
**D-5600 Wuppertal 1(DE)**

(54) **Neue Bicycloalkenyl-Prostaglandine und Verfahren zu ihrer Herstellung und ihre Verwendung als Arzneimittel.**

(57) Die vorliegende Erfindung betrifft neue Bicycloalkenyl-Prostaglandine, ein Verfahren zu ihrer Herstellung sowie ihre Verwendung als Arzneimittel.

EP 0 002 215 A1

Croydon Printing Company Ltd.

BAYER AKTIENGESELLSCHAFT
Zentralbereich
Patente, Marken und Lizenzen

5090 Leverkusen, Bayerwerk
KS/by
Ia (Pha)

Neue Bicycloalkenyl-Prostaglandine und Verfahren zu ihrer Herstellung und ihre Verwendung als Arzneimittel

Die vorliegende Erfindung betrifft neue Bicycloalkenyl-Prostaglandine, ein Verfahren zu ihrer Herstellung sowie ihre Verwendung als Arzneimittel.

Prostaglandine sind bereits bekannt geworden (Deutsche Offenlegungsschrift 2 150 361).

Die physiologischen Wirkungen von Prostaglandinen sind jedoch sowohl in vitro als auch im Säugetierorganismus von kurzer Dauer, da sie schnell in pharmakologisch unwirksame Metaboliten umgewandelt werden. Darüber hinaus ist es nachteilig, daß die Prostaglandine neben der gewünschten Wirkung gleichzeitig eine Reihe von unerwünschten physiologischen Nebenwirkungen besitzen, die ihre Verwendung als Arzneimittel stark einschränken.

Die Erfindung betrifft neue Bicycloalkenyl-Prostaglandine der allgemeinen Formel (I)

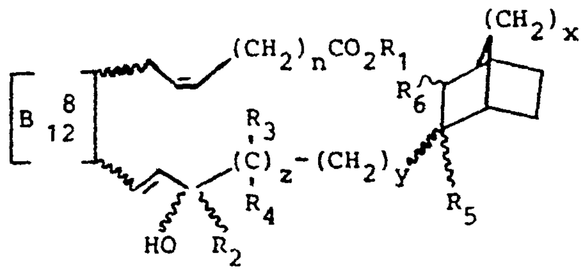

(I)

in welcher

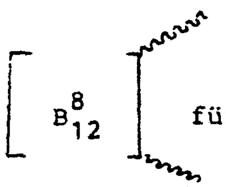

für die Teilstrukturen

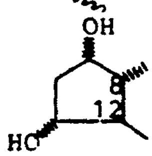

(Ia)

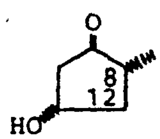

(Ib)

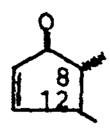

(Ic)

oder

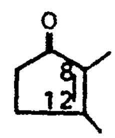

(Id)

steht,

$R_1$ für Wasserstoff oder einen gegebenenfalls substituierten Alkylrest steht,

$R_2$ für Wasserstoff oder einen Alkylrest steht

$R_3$ und $R_4$ gleich oder verschieden sind und für Wasserstoff oder einen gegebenenfalls substituierten Alkylrest oder Aralkylrest stehen, wobei einer der Substituenten immer Wasserstoff ist, wenn der andere für Aralkyl steht,

$R_5$ und $R_6$ gleich oder verschieden sind und für Wasserstoff oder einen gegebenenfalls substituierten Alkylrest oder Aralkylrest stehen,

z   für die Zahlen 0 bis 2 steht

y   für die Zahlen 0 bis 6 steht,

x   für die Zahlen 1 bis 4 steht,

n   für die Zahlen 2 bis 6 steht und

falls $R_1$ Wasserstoff bedeutet, auch deren physiologisch verträgliche Salze.

Die Schreibweise 〰 bedeutet, daß zum Beispiel die Substituenten $R_2$ und OH in α- oder ß-Position oder zum Beispiel die Substituenten $R_5$ und $R_6$ exo oder endo stehen können.

Die Schreibweise ıııııı bedeutet, daß die Substituenten α-ständig, die Schreibweise ——— bedeutet, daß die Substituenten ß-ständig angeordnet sind.

Die neuen Bicycloalkenyl-Prostaglandine können sowohl als Enantiomere, Enantiomerenpaare und Diastereomerenpaare vorliegen.

Es wurde gefunden, daß man die Bicycloalkenyl-Prostaglandine der allgemeinen Formel (I) erhält, wenn man gemäß dem Reaktionsschema (I) einen Aldehyd der allgemeinen Formel (II)

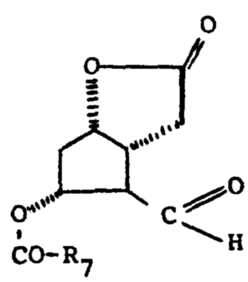

(II)

in welcher

$R_7$ für einen gegebenenfalls substituierten Alkyl- oder Arylrest steht, mit einen Phosphonester der allgemeinen Formel (III)

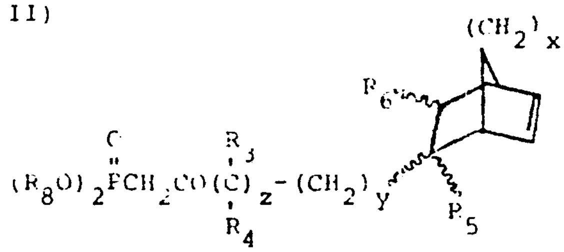

(III)

in welcher

$R_8$ für einen gegebenenfalls substituierten Alkylrest steht,

$R_3$, $R_4$, $R_5$, $R_6$, x, y und z die oben angegebene Bedeutung haben,

zu einen $\alpha$,ß-ungesättigten Keton der Formel (IV)

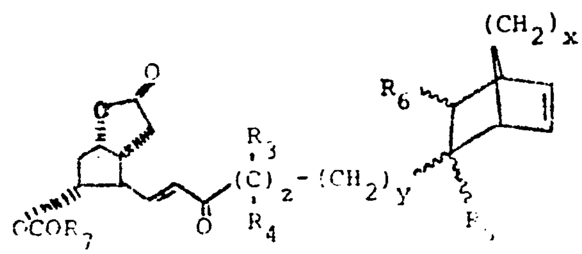

(IV)

in welcher

$R_3$, $R_4$, $R_5$, $R_6$, $R_7$, x, y und z die oben angegebene Bedeutung haben,

in Anwesenheit einer Base umsetzt und anschließend das $\alpha'$,ß-un-

gesättigte Keton (IV) mit einem komplexen Metallborhydrid, mit einem Aluminiumalkoholat oder gegebenenfalls mit einer metallorganischen Alkylverbindung zu einem Allylalkohol der allgemeinen Formel (V)

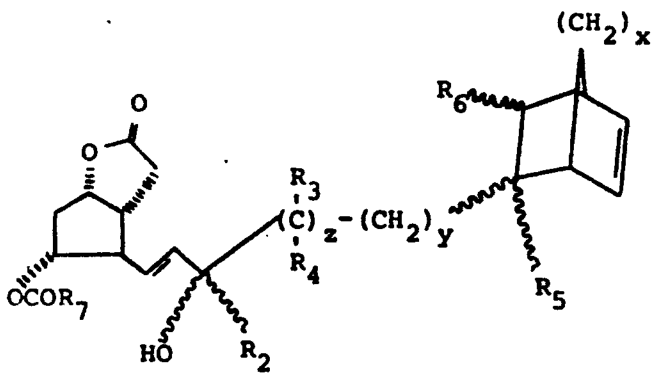

(V)

in welcher

$R_2, R_3, R_4, R_5, R_6, R_7$, x, y und z die oben aufgeführte Bedeutung haben,

reduziert, diesen durch Abspaltung der Gruppe $COR_7$ in ein Diol (VI) überführt, gegebenenfalls Schutzgruppen einführt und das so erhaltene Lacton der allgemeinen Formel (VII)

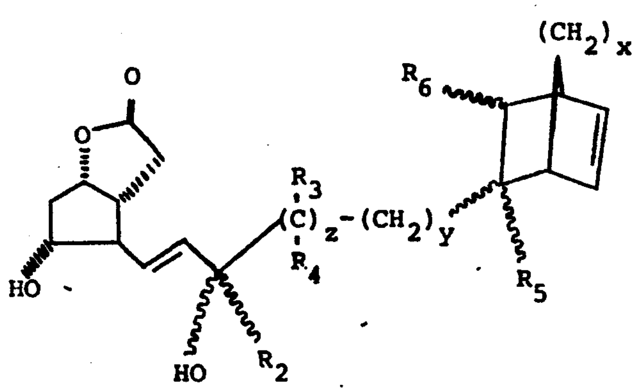

(VI)

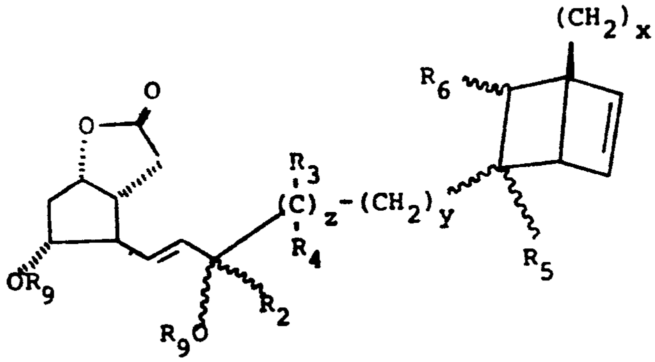

(VII)

in welcher

$R_9$ für einen Organosilyl- oder Ätherrest steht,

$R_2, R_3, R_4, R_5, R_6$, x, y und z die obige Bedeutung haben,

zu einem Lactol der allgemeinen Formel (VIII)

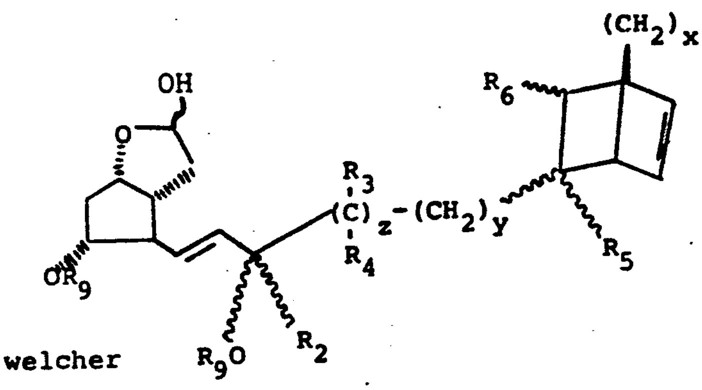

(VIII)

in welcher

$R_2, R_3, R_4, R_5, R_6, R_9$, x, y und z die obige Bedeutung haben,

reduziert und das so erhaltene Lactol mit einem Phosphoniumsalz der allgemeinen Formel (IX)

$$[(R_{10})_3{}^{\oplus}PCH_2(CH_2)_nCO_2H]\ X^{\ominus} \qquad (IX)$$

in welcher

$R_{10}$ für einen Arylrest,

X für ein Halogen und

n für eine Zahl zwischen 2 und 6 stehen,

in Anwesenheit einer Base zu einer Säure der allgemeinen Formel (X)

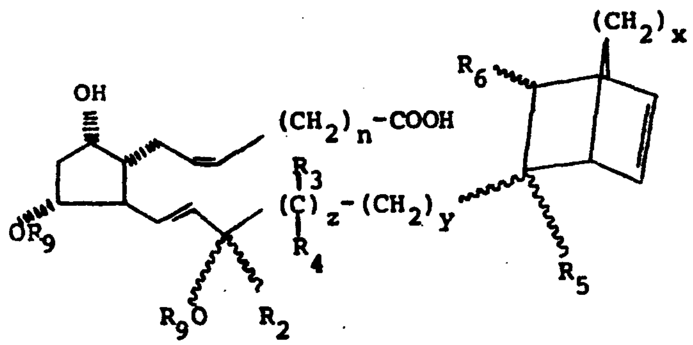

(X)

in welcher

$R_2, R_3, R_4, R_5, R_6, R_9$, n, y, x und z die obige Bedeutung haben,

umsetzt und anschließend für den Fall, daß in der Verbindung der allgemeinen Formel (I)

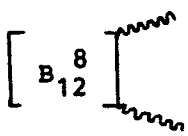 für 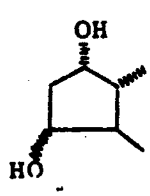 steht,

mit einer Säure gemäß dem Reaktionsschema I die eingeführten Schutzgruppen, gegebenenfalls unter Veresterung, wieder abspaltet und so Verbindungen mit der allgemeinen Formel (Ia) erhält,

oder gemäß dem Reaktionsschema II für den Fall, daß in der Verbindung der allgemeinen Formel (I)

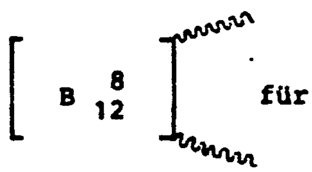 für 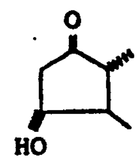 steht,

(X) zum Keton der allgemeinen Formel (XI)

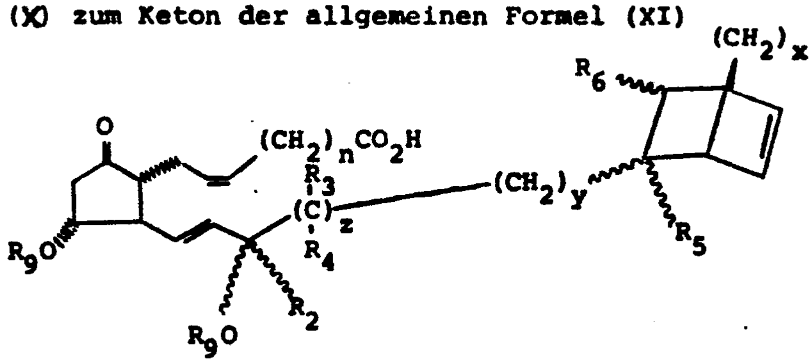

(XI)

in welcher

$R_2, R_3, R_4, R_5, R_6, R_9$, n, x, y und z die obige Bedeutung haben,

oxidiert und anschließend die Schutzgruppen, gegebenenfalls unter Veresterung abspaltet und so Verbindungen der allgemeinen Formel (Ib) erhält,

oder gemäß dem Reaktionsschema II für den Fall, daß in der Verbindung der allgemeinen Formel (I)

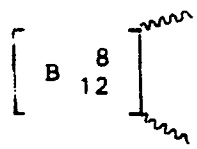 für 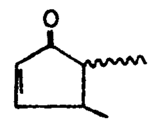 steht,

die Verbindung (Ib) oder (XI) mit einer Säure umsetzt und so die Verbindung der allgemeinen Formel (Ic) erhält,

oder gemäß dem Reaktionsschema II für den Fall, daß in der Verbindung der allgemeinen Formel (I)

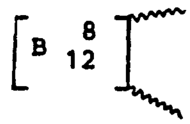 für 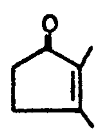 steht,

die Verbindung (Ib) mit einer Base umsetzt und so die Verbindung der allgemeinen Formel (Id) erhält.

Reaktionsschema I

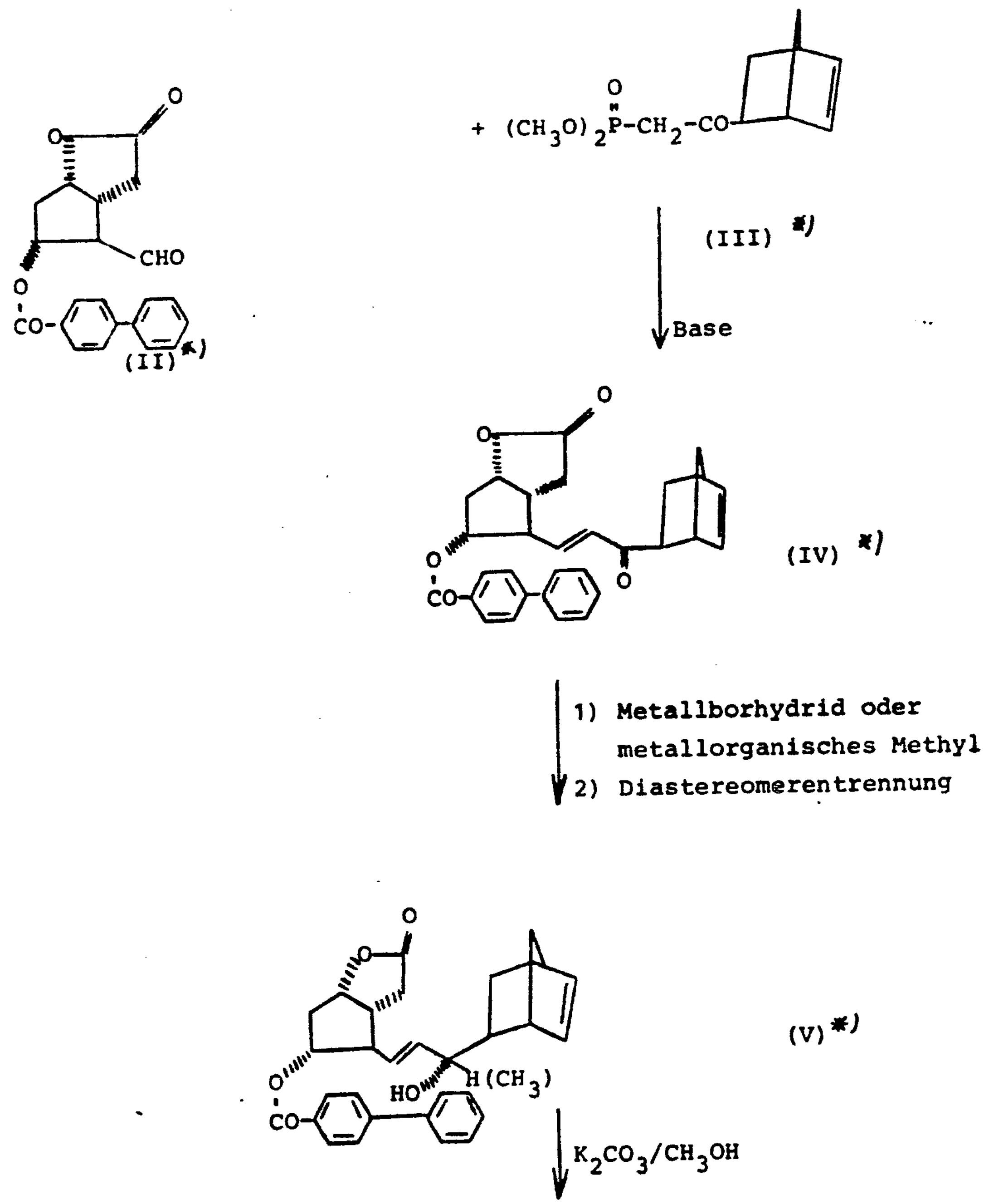


(II)*)

(III)*)

(IV)*)

(V)*)

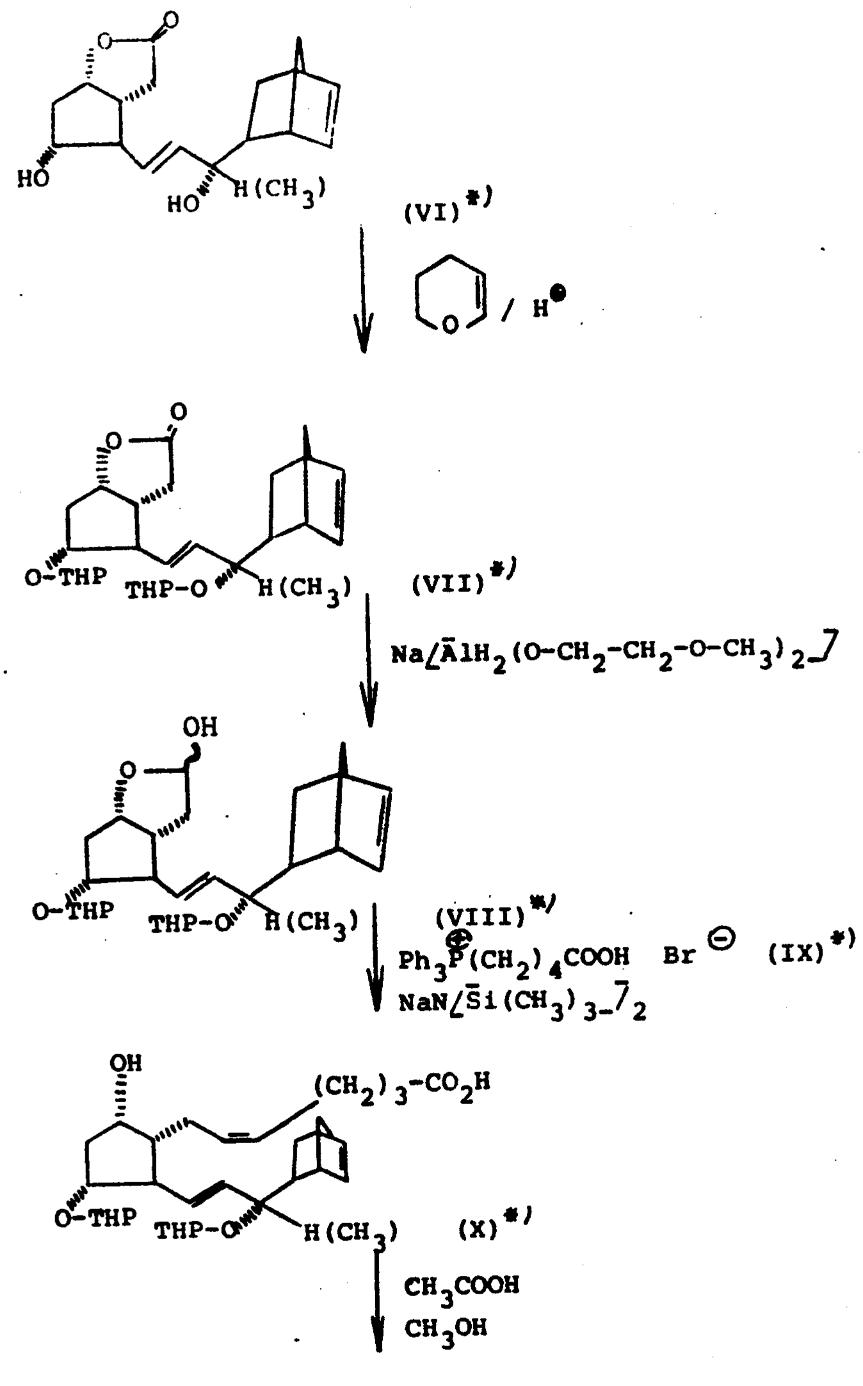
(VI)*)
/ H⊕
(VII)*)
Na/ĀlH2(O-CH2-CH2-O-CH3)2/
(VIII)*)
Ph3P⊕(CH2)4COOH Br⊖ (IX)*)
NaN/S̄i(CH3)3/2
(X)*)
CH3COOH
CH3OH

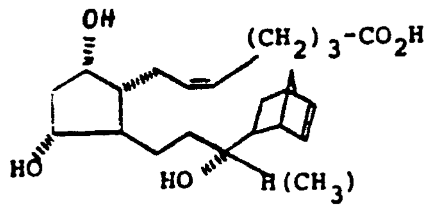

(Ia)*)

*) bedeutet Auswahl aus der römisch bezifferten allgemeinen Formel

THP ≙ 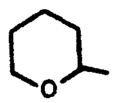

Reaktionsschema II

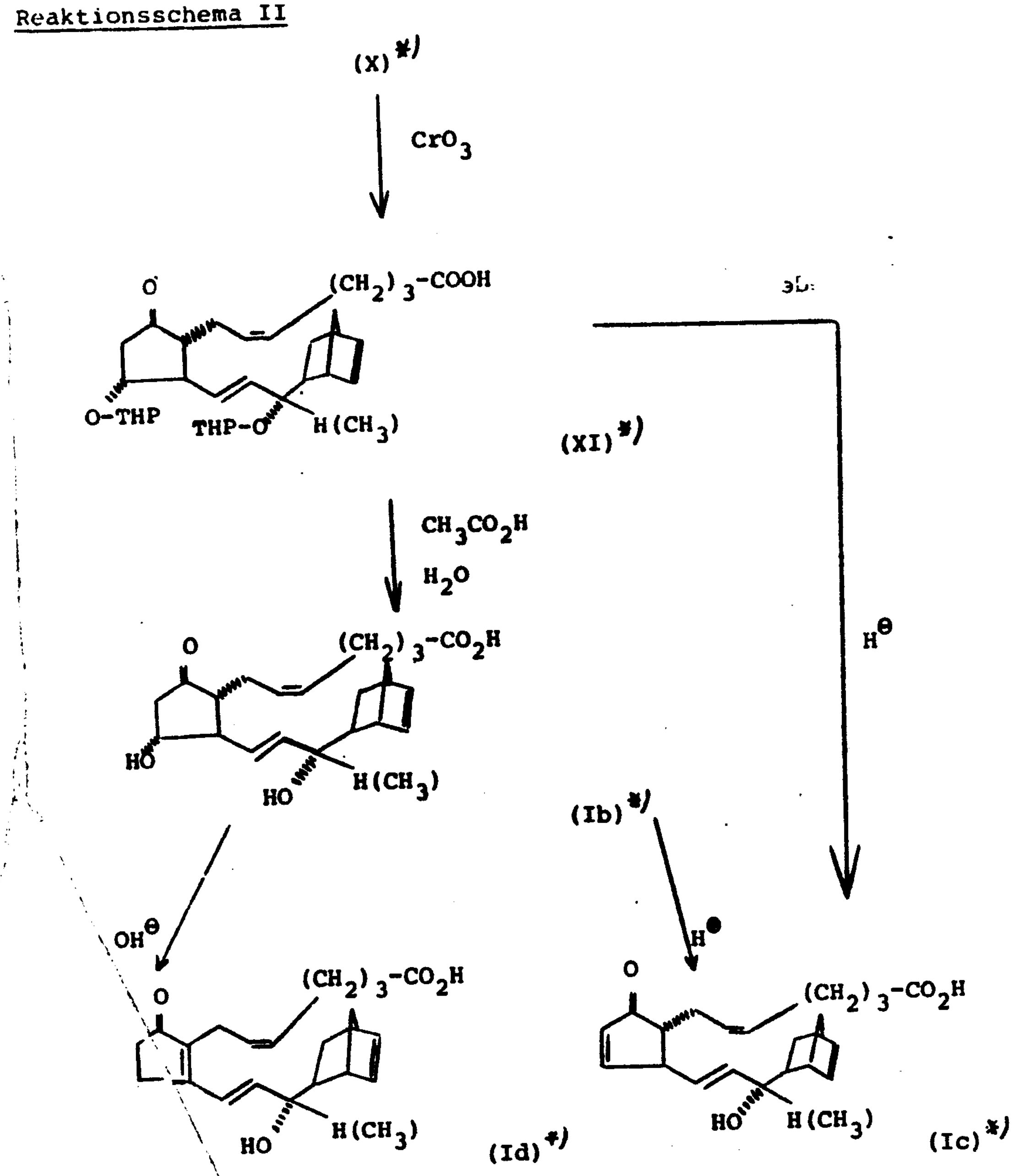


THP ≙ 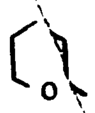

*) bedeutet Auswahl aus der römisch bezifferten allgemeinen Formel.

Entsprechend dem Reaktionsschema I wird in der ersten Stufe des Verfahrens ein Aldehyd der allgemeinen Formel (II) mit einem Phosphonester der allgemeinen Formel (III) in Gegenwart einer Base umgesetzt.

Die als Ausgangsstoffe verwendeten Aldehyde (II) sind bekannt oder können nach bekannten Verfahren hergestellt werden.

(J.Am.Chem.Soc. 1969, Bd. 91, Seite 5675,
J.Am.Chem.Soc. 1970, Bd. 92, Seite 397,
J.Am.Chem.Soc. 1971, Bd. 93, Seite 1491).

In der Formel (II) steht $R_7$ vorzugsweise für Alkyl mit 1-4 Kohlenstoffatomen, insbesondere für Methyl und vorzugsweise für Aryl, insbesondere Phenyl und p-Diphenyl. Beispielsweise seien folgende Verbindungen genannt:
2-Oxa-3-oxo-6-formyl-7-(acetyloxy-bicyclo/3,3,0/octan,
2-Oxa-3-oco-6-formyl-7-(p-phenylbenzoyloxy)-bicyclo/3,3,0/octan,
2-Oxa-3-oxo-6-formyl-7-(benzoyloxy)-bicyclo/3,3,0/octan.

Die weiterhin als Ausgangsstoffe verwendeten Phosphonester der allgemeinen Formel (III) sind bislang noch nicht bekannt, können aber nach grundsätzlich bekannten Verfahren hergestellt werden.
(J.Am.Chem.Soc. 1966, Bd. 88, Seite 1966).

In der Formel (III) stehen vorzugsweise
$R_3$,$R_4$,$R_5$ und $R_6$ für Wasserstoff, Benzyl und Alkyl mit 1 bis 4 Kohlenstoffatomen, insbesondere für Wasserstoff und Alkyl mit 1 bis 2 Kohlenstoffatomen,
$R_8$ für einen Alkylrest mit 1 bis 4 Kohlenstoffatomen, insbesondere für einen Alkylrest mit 1 bis 2 Kohlenstoffatomen,
Z vorzugsweise für eine Zahl von 0 bis 1
y vorzugsweise für eine Zahl von 0 bis 5 und
x vorzugsweise für eine Zahl von 1 bis 3.

Beispielsweise seien folgende Verbindungen, die der allgemeinen Formel (III) entsprechen, genannt:

2-(Bicyclo[2,2,1]hept-5-en-2-yl)-2-oxo-äthanphosphonsäure-dimethylester,
3-(Bicyclo[2,2,1]hept-5-en-2-yl)-2-oxo-butan-phosphonsäure-dimethylester,
3-(Bicyclo[2,2,1]hept-5-en-2-yl)-3-methyl-2-oxo-butan-phosphonsäuredimethylester,
7-(Bicyclo[2,2,1]hept-5-en-2-yl)-2-oxo-heptan-phosphonsäure-diäthylester,
2-(3-Methyl-bicyclo[2,2,1]hept-5-en-2-yl)-2-oxo-äthanphosphonsäuredimethylester,
2-(2-Methyl-bicyclo[2,2,1]hept-5-en-2-yl)-2-oxo-äthanphosphonsäuredimethylester,
2-(Bicyclo[2,2,2]oct-5-en-2-yl)-2-oxo-äthan-phosphonsäure-dimethylester,
6-(Bicyclo[2,2,2]oct-5-en-2-yl)-3-methyl-2-oxo-hexanphosphonsäuredimethylester,
2-(Bicyclo[3,2,2]non-8-en-6-yl)-2-oxo-äthan-phosphonsäure-dimethylester,
2-(7-Methyl-bicyclo[3,2,2]non-8-en-6-yl)-2-oxo-äthan-phosphonsäuredimethylester,

Das erfindungsgemäße Verfahren wird in der ersten Stufe in Gegenwart eines für die Reaktionspartner inerten Lösungsmittels durchgeführt. Die Wahl des Lösungsmittels hängt von der gegebenenfalls verwendeten Base ab. Geeignete Lösungsmittel sind beispielsweise Äther wie Tetrahydrofuran, Diäthyläther oder Dimethoxyäthan, Nitrile wie Acetonitril oder Propionitril, Amide wie Dimethylformamid, Dimethylacetamid oder heterocyclische Verbindungen wie N-Methylpyrrolidon oder Phosphorsäure-Derivate wie Hexamethylphosphorsäuretriamid.

Als gegebenenfalls verwendete Base eignen sich metallorganische Verbindungen wie Butyllithium oder tert.-Butyllithium, vorzugsweise n-Butyllithium oder Metallhydride wie Lithiumhydrid, Natriumhydrid oder Calciumhydrid, vorzugsweise Natriumhydrid, Alkoxide wie Natriummethylat, Kaliummethylat, Natrium-tert.-butylat, Kalium-tert.-butylat, vorzugsweise Natrium- oder Kalium-tert.-butylat oder Metallamide wie Natriumamid, Kaliumamid, vorzugsweise Natriumamid.

Das erfindungsgemäße Verfahren wird in der ersten Stufe im Temperaturbereich -20°C und +50°C, vorzugsweise -15°C und 30°C durchgeführt.

Im allgemeinen setzt man 1 Mol der Verbindung (II) mit 1,0 bis 1,5 Mol, vorzugsweise 1,05 bis 1,3 Mol des Phosphonesters (III), der vorher mit 1,0 bis 1,2 Mol, vorzugsweise 1,05 bis 1,15 Mol Base umgesetzt wurde, um.

Die Reaktionszeit ist von der Temperatur abhängig und liegt im allgemeinen zwischen einer halben Stunde und 3 Stunden. Im allgemeinen wird das beschriebene Verfahren unter Normaldruck durchgeführt.

Entsprechend dem Reaktionsschema I wird in der zweiten Stufe des Verfahrens das erhaltene α,ß-ungesättigte Keton der allgemeinen Formel (IV) mit einem komplexen Metallborhydrid umgesetzt. Als komplexe Metallborhydride eignen sich Metallborhydride wie Natriumborhydrid, vorzugsweise Zinkborhydrid oder auch Metallorganylborhydride wie Lithium-tris-isoamylborhydrid, Lithium-perhydro-9b-boraphenalylhydrid, Lithium-9-tert.-butyl-9-borabicyclo[3,3,1]nonylhydrid, Lithium-diisopinocamphenyl-tert.-butylborhydrid, Lithium-2-thexyl-4,8-dimethyl-2-borabicyclo[3,3,1]nonylhydrid, Kalium-tris-sek-butyl-borhydrid (Kaliumselectrid).

Die Reaktion wird in Gegenwart eines für die Reaktionspartner inerten Lösungsmittels durchgeführt. Die Wahl des Lösungsmittels ist abhängig vom Reduktionsmittel. Als Lösungsmittel eignen sich beispielsweise Äther wie Diäthyläther, Tetrahydrofuran oder Dimethoxy-äthan oder Kohlenwasserstoffe wie Toluol oder auch Gemische der in Frage kommenden Lösungsmittel.

Die Temperatur liegt zwischen -130°C und +20°C, vorzugsweise zwischen -105°C und 0°C.

Im allgemeinen setzt man 1 Mol der Verbindung (IV) mit mindestens 1 Hydridäquivalent des Reduktionsmittels um. Ein Überschuß schadet nicht.

Beim Einsatz von Zinkborhydrid ist es nicht erforderlich, das Reduktionsmittel als solches einzusetzen. Es genügt, Zinkborhydrid aus Natriumborhydrid und Zinkchlorid herzustellen und diese, das Reduktionsmittel enthaltene Lösung einzusetzen.

Die Reaktionszeit ist von der Temperatur abhängig und liegt im allgemeinen zwischen 4 und 20 Stunden.

Reduziert man das α,ß-ungesättigte Keton der allgemeinen Formel (IV) mit Metallborhydriden wie Natriumborhydrid oder vorzugsweise mit Zinkborhydrid, so erhält man Diastereomerengemische, die gegebenenfalls durch Chromatographie aufgetrennt werden können.

Reduziert man das Keton (IV) mit Metallborhydriden wie Lithium-tris-isoamylborhydrid, Lithiumperhydro-9b-boraphenalyl-hydrid, Lithium-9-tert-butyl-9-borabicyclo[3,3,1]nonylhydrid, Lithium-diisopinocamphenyl-tert.-butylborhydrid, Lithium-2-thexyl-4,8-dimethyl-2-borabicyclo [3,3,1]nonylhydrid oder Kaliumselectrid, so kann gegebenenfalls auf die Diastereomerentrennung, beispielsweise durch Chromatographie, verzichtet werden, da vorwiegend bis nahezu ausschließlich nur ein Diasteromeres entsteht.

Das α,β-ungesättigte Keton der allgemeinen Formel (IV) kann auch mit Aluminiumalkoholaten reduziert werden. Es eignet sich vorzugsweise Aluminiumtri-isopropylat.

Die Reaktion wird in Gegenwart eines für die Reaktionspartner inerten Lösungsmittels durchgeführt. Als Lösungsmittel eignen sich Aromaten wie Benzol, Toluol, insbesondere Toluol. Die Temperatur liegt zwischen 60°C und 110°C, vorzugsweise zwischen 70-90°C.

Im allgemeinen setzt man 1 Mol der Verbindung (IV) mit mindestens 1 Mol des Reduktionsmittels um. Ein Überschuß schadet nicht. Die Reaktionszeit ist von der Temperatur abhängig und liegt im allgemeinen zwischen 3 und 10 Stunden.

In einer Verfahrensvariante wird das α,β-ungesättigte Keton der allgemeinen Formel (IV) mit einer metallorganischen Alkylverbindung umgesetzt.

Als metallorganische Alkylverbindungen eignen sich Grignard-Verbindungen mit einem Alkylrest von 1 bis 6 Kohlenstoffatomen, vorzugsweise mit 1 bis 2 Kohlenstoffatomen oder Lithiumalkylverbindungen mit einem Alkylrest von 1 bis 6 Kohlenstoffatomen, vorzugsweise mit 1 bis 2 Kohlenstoffatomen.

Insbesondere seien genannt:
Methylmagnesiumchlorid, Methylmagnesiumbromid, Methylmagnesiumjodid, Methyllithium, Äthylmagnesiumbromid.

Die Reaktion wird in Gegenwart eines für die Reaktionspartner inerten Lösungsmittels durchgeführt. Als Lösungsmittel eignen sich beispielsweise Äther wie Diäthyläther, Tetrahydrofuran oder Dimethoxyäthan oder Gemische der in Frage kommenden Lösungsmittel.

Die Temperaturen liegen zwischen -90°C und 0°C, vorzugsweise zwischen -78°C und -10°C.

Im allgemeinen setzt man 1 Mol der Verbindung (IV) mit 1,0 bis 3 Mol der metallorganischen Alkylverbindung, vorzugsweise mit 1,0 bis 2 Mol um. Die Reaktionsdauer ist von der Temperatur abhängig und liegt zwischen 1 bis 2 Stunden.

Das bei den Alkylierungen α,ß-ungesättigter Ketone der allgemeinen Formel (IV) mit metallorganischen Alkylverbindungen wie Methylmagnesiumjodid anfallende Diatereomerengemisch läßt sich gegebenenfalls durch Chromatographie auftrennen.

Entsprechend dem Reaktionsschema I wird in einer dritten Stufe des Verfahrens der Allylalkohol der allgemeinen Formel (V) mit Alkalihydroxiden oder Alkalicarbonaten in Wasser oder Alkoholen, vorzugsweise mit Alkalicarbonaten in Alkoholen umgesetzt. Als Alkalicarbonate eignen sich beispielsweise Kalium- oder Natriumcarbonat, insbesondere Kaliumcarbonat. Als Alkohole eignen sich vorzugsweise solche mit einem geradkettigen Alkylrest mit 1-4 Kohlenstoffatomen, insbesondere Methanol und Äthanol.

Das erfindungsgemäße Verfahren wird in der dritten Stufe im Temperaturbereich von 0°C und 50°C, vorzugsweise von 20°C und 30°C durchgeführt. Die Reaktionszeit ist von der Temperatur abhängig und liegt im allgemeinen zwischen einer Stunde

und drei Stunden.

Im allgemeinen setzt man 1 Mol des Allylalkohols der allgemeinen Formel (V) mit 1,0 bis 1,2 Mol Alkalicarbonat, vorzugsweise mit 1,0 bis 1,1 Mol um.

In einer vierten Stufe des erfindungsgemäßen Verfahrens führt man gegebenenfalls Schutzgruppen entsprechend dem Reaktionsschema I in das Diol der allgemeinen Formel (VI) ein. Zur Einführung von Schutzgruppen eignen sich acyclische wie cyclische Enoläther, beispielsweise Dihydropyran und Äthylvinyläther, insbesondere Dihydropyran.

Als Verdünnungsmittel kommen alle inerten organischen Lösungsmittel in Frage. Hierzu gehören vorzugsweise chlorierte Kohlenwasserstoffe wie Methylenchlorid, Chloroform und Äther wie Diäthyläther, Tetrahydrofuran oder Dimethoxyäthan.

Als Katalysatoren kommen alle üblichen Säuren in Frage. Hierzu gehören vorzugsweise Salzsäure, Schwefelsäure, Oxalsäure, p-Toluolsulfonsäure, insbesondere p-Toluolsulfonsäure.

Die Reaktionstemperaturen können über einen größeren Bereich variiert werden. Im allgemeinen arbeitet man zwischen etwa 0°C und 50°C, vorzugsweise zwischen 20°C und 30°C.

Im allgemeinen setzt man 1 Mol des Diols (VI) mit mindestens 2 Mol Enoläther um. Ein Überschuß schadet nicht.

In einer Verfahrensvariante wird das Diol der allgemeinen Formel (VI) mit Trialkylsilylhalogeniden in Anwesenheit eines Säurebinders umgesetzt.

Als Trialkylsilylhalogenide eignen sich Trimethylchlorsilan und tert-Butyl-dimethyl-chlorsilan, vorzugsweise tert-Butyl-dimethyl-chlorsilan.

Als Säurebinder können alle üblichen Säurebindungsmittel verwendet werden. Hierzu behören vorzugsweise aliphatische tertiäre Amine und aromatische N-Heterocyclen. Als besonders geeignet sei Imidazol genannt.

Als Lösungsmittel eignen sich beispielsweise aliphatische Amide wie Dimethylformamid, insbesondere Dimethylformamid oder auch Phosphorsäurederivate wie Dimethylacetamid, Hexamethylphosphorsäuretriamid.

Die Temperaturen liegen zwischen 10°C und 80°C, vorzugsweise zwischen 20°C und 50°C. Die Reaktionsdauer ist von der Temperatur abhängig und liegt zwischen 6 und 24 Stunden.

Bei der Durchführung des erfindungsgemäßen Verfahrens setzt man 1 Mol des Diols (VI) mit 2,2 bis 4,0 Mol, vorzugsweise 2,4 bis 3,0 Mol tert-Butyl-dimethyl-chlor-silan in Anwesenheit von 4,4 bis 8,0 Mol, vorzugsweise 5,0 bis 7,0 Mol Imidazol um.

In einer vierten Stufe des erfindungsgemäßen Verfahrens reduziert man gemäß Reaktionsschema I das Lacton der allgemeinen

Formel (VII) mit Alkali-alkoxy-hydrido-aluminaten, beispielsweise Natrium-bis-(2-methoxy-äthoxy)-dihydrido-aluminat oder Natrium-äthoxy-bis-(2-methoxy-äthoxy)-hydrido-aluminat.

Ein besonders geeignetes Reduktionsmittel ist das Natrium-bis-(2-methoxy-äthoxy)-dihydridoaluminat. Als Verdünnungsmittel eignet sich Toluol. Die Reaktionstemperaturen liegen zwischen -78°C und -40°C, vorzugsweise zwischen -78°C und -70°C. Die Reaktionszeit hängt von der Reaktionstemperatur ab und liegt zwischen vier und zehn Stunden. Im allgemeinen setzt man 1 Mol des Lactons (VII) mit mindestens einem Hydridäquivalent des Reduktionsmittels um. Ein Überschuß schadet nicht.

In einer fünften Stufe des erfindungsgemäßen Verfahrens wird entsprechend dem Reaktionsschema I das Lactol der allgemeinen Formel (VIII) mit einem Phosphoniumsalz der allgemeinen Formel (IX) in Gegenwart einer Base umgesetzt.

Die als Ausgangsstoffe verwendeten Phosphoniumsalze sind bekannt (J.Am.Chem.Soc. 1969, Bd. 91, Seite 5675).

In der Formel (IX) steht n vorzugsweise für 2 bis 4 und x für Cl, Br, j, insbesondere für Br und Cl.

Beispielsweise seien folgende Verbindungen genannt:
3-Carboxy-propyl-triphenylphosphoniumbromid,
4-Carboxy-butyl-triphenylphosphoniumbromid,
5-Carboxy-pentyl-triphenylphosphoniumbormid,
4-Carboxy-butyl-triphenylphosphoniumchlorid.

Als gegebenenfalls verwendete Base eignen sich Alkalihydride, wie Natriumhydrid, Kaliumhydrid, vorzugsweise Natriumhydrid, Alkali-bis-(trialkylsilyl)amide wie Natrium-bis-(trialkylsilyl)-amide mit einem Alkyl von 1-4 Kohlenstoffatomen, insbesondere Natrium-bis(trimethylsilyl)-amid.

Die Wahl des Lösungsmittels hängt von der gegebenenfalls verwendeten Base ab. Verwendet man Alkali-bis-(trialkylsilyl)-amide als Base, so führt man die Reaktion in einem inerten Lösungsmittel wie Äther, beispielsweise Diäthyläther, Tetrahydrofuran, Dialkoxyäthan, insbesondere Dimethoxyäthan durch. Die Reaktionstemperatur liegt in einem Temperaturbereich von 0°C und 40°C, vorzugsweise von 5°C und 30°C.

Die Reaktionszeit ist von dem Temperaturbereich abhängig und liegt im allgemeinen zwischen einer Stunde und 4 Stunden.

Verwendet man Alkalihydrid als Base, so führt man die Reaktion im Dimethylsulfoxid als Lösungsmittel durch.

Die Reaktionstemperatur liegt zunächst in einem Temperaturbereich zwischen 30°C und 90°C, vorzugsweise von 60°C und 70°C (Herstellung des Natriumsalzes des Dimethylsulfoxids) und anschließend in einem Temperaturbereich zwischen 10°C und 40°C, vorzugsweise 15°C und 25°C. Die Reaktionszeit ist von der Temperatur abhängig und liegt im allgemeinen zwischen einer Stunde und zwei Stunden.

Im allgemeinen setzt man 1 Mol des Lactols (VIII) mit 1,0 bis 3,0 Mol, vorzugsweise 2,0 bis 3,0 Mol eines Phosphoniumsalzes der allgemeinen Formel (IX) um, das zunächst mit 2,0 bis 6,5 Mol, vorzugsweise 2,6 bis 6,1 Mol Base umgesetzt wurde.

In einer sechsten Stufe des erfindungsgemäßen Verfahrens wird entsprechend dem Reaktionsschema I die Verbindung der allgemeinen Formel (X) mit einer Säure behandelt.

Als Säuren eignen sich niedere aliphatische Mono- und Dicarbonsäuren oder auch niedere aliphatische Hydroxytri-carbonsäuren. Beispielsweise seien genannt:

Essigsäure, Propionsäure, Oxalsäure, Zitronensäure. Vorzugsweise verwendet man Essigsäure. Die Reaktion wird in einem organischen Lösungsmittel in Gegenwart von Wasser durchgeführt. Es eignen sich solche Lösungsmittel, die mit Wasser mischbar und gegenüber den eingesetzten Säuren inert sind. Es seien beispielsweise genannt:

Tetrahydrofuran oder Dioxan.
Als Lösungsmittel eignen sich auch gegebenenfalls die verwendeten Säuren, beispielsweise Essigsäure. Vorzugsweise werden Gemische aus Wasser, niederer aliphatischer Säure und organischem Lösungsmittel verwendet. Als Lösungsmittel eignen sich weiter niedere Alkohole wie Methanol, Äthanol, Propanol, vorzugsweise Methanol, die auch gegebenenfalls ohne Wasser verwendet werden können.

Die Zusammensetzung des Gemisches ist nicht kritisch, die Verbindung (X) soll jedoch darin löslich sein. Zweckmäßig verwendet man einen größeren Überschuß an Säure. Die Temperatur liegt zwischen +20°C und +60°C, vorzugsweise bei +30°C bis +55°C. Die Reaktionsdauer ist von der Temperatur abhängig und liegt zwischen 2 und 10 Stunden.

Als neue Verbindungen (Ia) gemäß Reaktionsschema I seien beispielhaft genannt:

(5Z, 9α, 11α, 13E, 15α)-9,11,15-Trihydroxy-15-(bicyclo[2,2,1]hept-5-en-2-yl)-16,17,18,19,20-penta-nor-prosta-5,13-diensäure,

(5Z, 9α, 11α, 13E, 15α)-9,11,15-Trihydroxy-16-(bicyclo[2,2,1]hept-5-en-2-yl)-18,19,20-tri-nor-prosta-5,13-diensäure,

(5Z, 9α, 11α, 13E, 15α)-9,11,15-Trihydroxy-16-(bicyclo[2,2,1]hept-5-en-2-yl)-16-methyl-18,19,20-tri-nor-prosta-5,13-diensäure,

(5Z, 9α, 11α, 13E, 15α)-9,11,15-Trihydroxy-20-(bicyclo[2,2,1]hept-5-en-2-yl)-prosta-5,13-diensäure,

(5Z, 9α, 11α, 13 E, 15α)-9,11,15-Trihydroxy-15-(3-methyl-bicyclo[2,2,1]hept-5-en-2-yl)-16,17,18,19,20-penta-nor-prosta-5,13-diensäure,

(5Z, 9α, 11α, 13E, 15α)-9,11,15-Trihydroxy-15-(2-methyl-bicyclo[2,2,1]hept-5-en-2-yl)-16,17,18,19,20-penta-nor-prosta-5,13-diensäure.

(5Z, 9α, 11α, 13 E, 15α)-9,11,15-Trihydroxy-15ß-methyl-20(bicyclo[2,2,1]hept-5-en-2-yl)-prosta-5,13-diensäure,

(5Z, 9α, 11α, 13E, 15α)-9,11,15-Trihydroxy-15-(bicyclo[2,2,2]oct-5-en-2-yl)-16,17,18,19,20-penta-nor-prosta-5,13-diensäure,

(5Z, 9α, 11α, 13E, 15α)-9,11,15-Trihydroxy-19-(bicyclo-[2,2,2]oct-5-en-2-yl)-16-methyl-20-nor-prosta-5,13-diensäure,

(5Z, 9α, 11α, 13E, 15α)-9,11,15-Trihydroxy-15-(bicyclo[3,2,2]non-8-en-6-yl)-16,17,18,19,20-penta-nor-prosta-5,13-diensäure,

(5Z, 9α, 11α, 13E, 15α)-9,11,15-Trihydroxy-15-(7-methyl-bicyclo[3,2,2]non-8-en-6-yl)-16,17,18,19,20-penta-nor-prosta-5,13-diensäure,

ihre 15ß-Diastereomeren bzw. nach bekannten Verfahren hergestellten entsprechenden Ester. Die Verbindungen können bezüglich ihrer Konfiguration am bicyclischen Ringsystem in der exo- oder endo-Form vorliegen.

Entsprechend dem Reaktionsschema II wird in einer weiteren Stufe des erfindungsgemäßen Verfahrens eine Verbindung der allgemeinen Formel (X) mit sechswertigem Chrom oxidiert.

Ein geeignetes Oxidationsmittel ist das Jones-Reagens, das heißt angesäuerte Chromsäure (Journal of the Chemical Society 1947, Seite 39). Als Verdünnungsmittel eignet sich Aceton. Zweckmäßig setzt man 1 Mol der Verbindung mit der stöchiometrisch berechneten Menge bzw. mit einem bis zu 4-molaren Überschuß an Oxidationsmittel um. Die Reaktionstemperaturen liegen zwischen -70°C und +20°C, zweckmäßig bei -20°C bis 0°C.

Ebenso geeignet für die obige Reaktion ist das Collins-Reagens, d.h. Chromtrioxid in Pyridin (Tetrahedron Letters 1968, Seite 3363). Als Verdünnungsmittel eignet sich Methylenchlorid. Zweckmäßig setzt man 1 Mol der Verbindung mit dem 5- bis 10-fachen Überschuß, vorzugsweise mit dem 6- bis 8-fachen Überschuß der Oxidation einer sekundären Hydroxylgruppe an Oxidationsmittel um. Die Reaktionstemperatur liegt zwischen -20°C bis +30°C, vorzugsweise zwischen -10°C bis +10°C.

In einer weiteren Stufe des erfindungsgemäßen Verfahrens wird entsprechend Reaktionsschema II die Verbindung der allgemeinen Formel (XI) mit einer Säure behandelt.

Als Säuren eignen sich niedere aliphatische Mono- und Dicarbonsäuren oder auch niedere aliphatische Hydroxy-tricarbonsäuren; beispielsweise seien genannt:

Essigsäure, Propionsäure, Oxalsäure, Zitronensäure. Vorzugsweise verwendet man Essigsäure. Die Reaktion wird in einem organischen Lösungsmittel in Gegenwart von Wasser durchgeführt. Es eignen sich solche Lösungsmittel, die mit Wasser mischbar und gegenüber den eingesetzten Säuren inert sind. Es seien beispielsweise genannt:

Tetrahydrofuran oder Dioxan. Als Lösungsmittel eignen sich auch gegebenenfalls die verwendeten Säuren, beispielsweise Essigsäure. Vorzugsweise werden Gemische aus Wasser, niederer aliphatischer Säure und organischem Lösungsmittel verwendet.

Als Lösungsmittel eignen isch weiter niedere Alkohole wie Methanol, Äthanol, Propanol, vorzugsweise Methanol.

Die Zusammensetzung des Gemisches ist nicht kritisch, die Verbindung (XI) soll jedoch darin löslich sein. Zweckmäßig verwendet man einen größeren Überschuß an Säure. Die Temperatur liegt zwischen +20°C bis +60°C, vorzugsweise bei +30°C bis +55°C. Die Reaktionsdauer ist von der Temperatur abhängig und liegt zwischen 2 bis 10 Stunden.

Als neue erhaltene Verbindungen gemäß Reaktionsschema II seien beispielhaft genannt:

(5Z, 11α, 13E, 15α)-9-Oxo-11,15-dihydroxy-15-(bicyclo [2,2,1]hept-5-en-2-yl)-16,17,18,19,20-penta-nor-prosta-5,13-diensäure,

(5Z, 11α, 13E, 15α)-9-Oxo-11,15-dihydroxy-16-(bicyclo [2,2,1]hept-5-en-2-yl)-18,19,20-tri-nor-prosta,5,13-diensäure,

(5Z, 11α, 13E, 15α)-9-Oxo-11,15-dihydroxy-16-(bicyclo [2,2,1]hept-5-en-2-yl)-16-methyl-18,19,20-tri-nor-prosta-5,13-diensäure,

(5Z, 11α, 13E, 15α)-9-Oxo-11,15-dihydroxy-20-(bicyclo [2,2,1]hept-5-en-2-yl)-prosta-5,13-diensäure,

(5Z, 11α, 13E, 15α)-9-Oco-11,15-dihydroxy-15-(3-methyl-bicyclo[2,2,1]hept-5-en-2-yl)-16,17,18,19,20-penta-nor-prosta-5,13-diensäure,

(5Z, 11α, 13E, 15α)-9-Oxo-11,15-dihydroxy-15-(2-methyl-bicyclo[2,2,1]hept-5-en-2-yl)-16,17,18,19,20-penta-nor-prosta-5,13-diensäure,

(5Z, 11α, 13E, 15α)-9-Oxo-11,15-dihydroxy-15ß-methyl-20-(bicyclo[2,2,1]hept-5-en-2-yl)-prosta-5,13-diensäure,

(5Z, 11α, 13E, 15α)-9-Oxo-11,15-dihydroxy-15-(bicyclo[2,2,2]oct-5-en-2-yl)-16,17,18,19,20-penta-nor-prosta-5,13-diensäure,

(5Z, 11α, 13E, 15α)-9-Oxo-11,15-dihydroxy-19-(bicyclo[2,2,2]oct-5-en-2-yl)-16-methyl-20-nor-prosta-5,13-diensäure,

(5Z, 11α, 13E, 15α)-9-Oxo-11,15-dihydroxy-15(bicyclo[3,2,2]non-8-en-6-yl)-16,17,18,19,20-penta-nor-prosta-5,13-diensäure,

(5Z, 11α, 13E, 15α)-9-Oxo-11,15-dihydroxy-15-(7-methyl-bicyclo[3,2,2]non-8-en-6-yl)-16,17,18,19,20-penta-nor-prosta-5,13-diensäure

ihre 15ß-Diastereomeren bzw. die nach bekannten Verfahren hergestellten entsprechenden Ester. Die Verbindungen können bezüglich ihrer Konfiguration am bicyclischen Ringsystem entweder in der exo- oder endo-Form vorliegen.

In einer weiteren Stufe des erfindungsgemäßen Verfahrens wird entsprechend Reaktionsschema II die Verbindung der allgemeinen Formel (Ib) mit einer Säure behandelt.

Als Säuren eignen sich anorganische Säuren wie Salzsäure, Schwefelsäure, Phosphorsäure, vorzugsweise sei genannt Salzsäure, organische Säuren wie Sulfonsäuren, beispielsweise Methansulfonsäure, Benzolsulfonsäure, p-Toluolsulfonsäure, vorzugsweise p-Toluolsulfonsäure.

Als Verdünnungsmittel kommen solche Lösungsmittel in Frage, die mit Wasser mischbar sind und gegenüber den eingesetzten Säuren stabil sind. Es seien beispielsweise genannt: Tetrahydrofuran oder Dioxan.

Als Lösungsmittel eignen sich weiter niedere Alkohole, wie Methanol, Äthanol, vorzugsweise Methanol, die bei Abwesenheit von Wasser gegebenenfalls die Ester der Verbindungen der allgemeinen Formel (Ic) liefern.

Die Zusammensetzung des Gemisches ist nicht kritisch, jedoch soll die Verbindung (Ib) darin löslich sein.

Die verwendete Säuremenge richtet sich nach dem pH-Bereich. Der pH-Bereich soll 1 bis 3,5 - vorzugsweise 1,5 bis 2 betragen. Die Temperatur liegt zwischen +10°C und +35°C, vorzugsweise zwischen +20°C und +30°C. Die Reaktionsdauer ist von der Temperatur und dem pH abhängig und liegt zwischen 3 Stunden und 4 Tagen.

Weiterhin lassen sich nach einer Verfahrensvariante Verbindungen der allgemeinen Formel (Ic) direkt aus Verbindungen der allgemeinen Formel (XI) gemäß Reaktionsschema II durch Behandlung mit Säuren herstellen.

(5Z, 13E, 15α)-9-Oxo-15-hydroxy-15-(bicyclo/2,2,1/hept-5-en-2-yl)-16,17,18,19,20-penta-nor-prosta-5,10,13-triensäure,

(5Z, 13E, 15α)-9-Oxo-15-hydroxy-16-(bicyclo/2,2,1/hept-5-en-2-yl)-18,19,20-tri-nor-prosta-5,10,13-triensäure,

(5Z, 13E, 15α)-9-Oxo-15-hydroxy-16(bicyclo/2,2,1/hept-5-en-2-yl)-16-methyl-18,19,20-tri-nor-prosto-5,10,13-triensäure,

(5Z, 13E, 15α)-9-Oxo-15-hydroxy-20-(bicyclo/2,2,1/hept-2-en-5-yl)-prosta-5,10,13-triensäure,

(5Z, 13E, 15α)-9-Oxo-15-hydroxy-15(3-methyl-bicyclo /2,2,1/hept-5-en-2-yl)-16,17,18,19,20-penta-nor-prosta-5,10,13-triensäure,

(5Z, 13E, 15α)-9-Oxo-15-hydroxy-15-(2-methyl-bicyclo /2,2,1/hept-5-en-2-yl)-16,17,18,19,20-penta-nor-prosta-5,10,13-triensäure,

(5Z, 13E , 15α)-9-Oxo-15-hydroxy-15ß-methyl-20-(bicyclo /2,2,1/hept-5-en-2-yl)-prosta-5,10,13-triensäure,

(5Z, 13E, 15α)-9-Oxo-15-hydroxy-15(bicyclo/2,2,2/oct-5-en-2-yl)-16,17,18,19,20-penta-nor-prosta-5,10,13-triensäure,

(5Z, 13E, 15α)-9-Oxo-15-hydroxy-19-(bicyclo/2,2,2/oct-5-en-2-yl)-16-methyl-20-nor-prosta-5,10,13-triensäure,

(5Z, 13E, 15α)-9-Oxo-15-hydroxy-15-(bicyclo/3,2,2/non-8-en-6-yl)-16,17,18,19,20-penta-nor-prosta-5,10,13-triensäure,

(5Z, 13E, 15α)-9-Oxo-15-hydroxy-15-(7-methyl-bicyclo[3,2,2]non-8-en-6-yl)-16,17,18,19,20-penta-nor-prosta-5,10,13-triensäure,

ihre 15ß-Diastereomeren bzw. die nach bekannten Verfahren hergestellten entsprechenden Ester. Die Verbindungen können bezüglich ihrer Konfiguration am bicyclischen Ringsystem in der exo- oder endo-Form vorliegen.

Entsprechend dem Reaktionsschema II erhält man in einer weiteren Stufe des erfindungsgemäßen Verfahrens die Verbindungen der allgemeinen Formel (Id), wenn man die Verbindungen der allgemeinen Formel (Ib) mit Basen behandelt.

Als Basen eignen sich beispielsweise Alkali- und Erdalkalihydroxide wie Natriumhydroxid, Kaliumhydroxid, Bariumhydroxid, vorzugsweise Kaliumhydroxid, Alkali- und Erdalkalialkoholate wie Natriummethylat, Natriumäthylat, Kalium-tert.-butylat, Magnesiumäthylat, vorzugsweise Natriummethylat, aliphatische und cycloaliphatische Amine und Amidine wie Triäthylamin und 1,8-Diazabicyclo[5,4,0]undec-7-en (DBU), vorzugsweise DBU.

Die Reaktion wird in einem Lösungsmittel durchgeführt. Als Lösungsmittel eignen sich beispielsweise Wasser, niedrige aliphatische Alkohole, insbesondere Methanol und Äthanol.

Die Temperaturen liegen zwischen 0°C und +40°C, vorzugsweise zwischen +10°C und +30°C.

Die verwendete Basenmenge richtet sich nach dem pH-Bereich. Der pH-Bereich liegt bei 7 bis 12, vorzugsweise bei 9 bis 11.

Die Reaktionsdauer ist von der Temperatur und vom pH abhängig und liegt zwischen 1 und 5 Stunden.

Als neue Verbindungen erhalten gemäß Reaktionsschema II seien beispielhaft genannt:

(5Z, 13E, 15α)-9-Oxo-15-hydroxy-15-(bicyclo/2,2,1/hept-5-en-2-yl-16,17,18,19,20-penta-nor-prosta-5,8,13-triensäure,

(5Z, 13E, 15α)-9-Oxo-15-hydroxy-20-(bicyclo/2,2,1/hept-5-en-2-yl)-prosta-5,8,13-triensäure,

(5Z, 13E, 15α)-9-Oxo-15-hydroxy-19-(bicyclo/2,2,2/oct-5-en-2-yl)-16-methyl-20-nor-prosta-5,8,13-triensäure,

ihre 15β-Diastereomeren bzw. die nach bekannten Verfahren hergestellten entsprechenden Ester. Die Verbindungen können bezüglich ihrer Konfiguration am bicyclischen Ringsystem in der exo- oder endo-Form vorliegen.

Die erfindungsgemäßen neuen Bicycloalkenyl-Prostaglandine der allgemeinen Formel (I) haben im Vergleich zu den natürlichen Prostaglandinen differenzierte pharmakologische Wirkungen. Sie sind wertvolle Pharmaka, da sie in der Wirkung spezifischer sind, d.h., ein engeres biologisches Spektrum besitzen, geringere unerwünschte Nebenwirkungen verursachen und in der erwünschten Wirkung länger anhalten.

Sie eignen sich beispielsweise zur Behandlung von Asthma, zur Herabsetzung einer übermäßigen Magensaftsekretion, als Blutdrucksenker, Antithrombotika oder als Diuretika, als Arzneimittel zur Einleitung von Geburten oder auch als Kontraceptiva

zur Anwendung beim Menschen als auch zur Brunstsynchronisation bei verschiedenen Tierarten, beispielsweise Pferden, Rindern oder Schweinen, schließlich auch zur Beeinflussung der Arteriosklerose.

Die neuen erfindungsgemäßen Verbindungen können je nach Verwendungsart beispielsweise intravenös, intramuskulär, subkutan, oral oder intravaginal verabreicht werden.

Von besonderem Interesse sind Verbindungen der allgemeinen Formel (I) in welcher

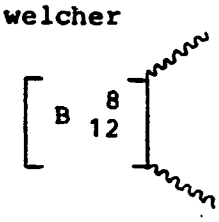

für die obengenannten Teilstrukturen steht,

$R_1$ für Wasserstoff oder Alkyl mit 1 bis 4 Kohlenstoffatomen steht,

$R_2$ für Wasserstoff oder Alkyl mit 1 bis 4, insbesondere 1 oder 2 Kohlenstoffatomen steht,

$R_3$ und $R_4$ gleich oder verschieden sind und jeweils für Wasserstoff oder Alkyl mit 1 bis 4, insbesondere 1 oder 2 Kohlenstoffatomen stehen,

$R_5$ und $R_6$ gleich oder verschieden sind und jeweils für Wasserstoff oder einen Alkylrest mit 1 bis 4, insbesondere 1 oder 2 Kohlenstoffatomen stehen,

z für O, 1 oder 2 steht,

y für eine ganze Zahl von O bis 6, insbesondere von O bis 4, steht,

x für eine ganze Zahl von 1 bis 4 steht und

n für eine ganze Zahl von 2 bis 6, insbesondere von 3 bis 5 steht,

und falls $R_1$ Wasserstoff bedeutet, auch deren physiologisch verträglichen Salze.

Als Zubereitungsform kommen die üblichen galenischen Applikationsformen in Frage, beispielsweise Infusions- oder Injektionslösungen, Tabletten, Cremes, Emulsionen, Suppositorien oder Aerosole.

Die neuen erfindungsgemäßen Verbindungen können als freie Säuren, als Ester oder in Form ihrer physiologisch unbedenklichen anorganischen oder organischen Salze angewendet werden.

Als Salze kommen beispielsweise in Frage:

Alkalisalze, Triäthylammonium-, Benzylammonium-, Morpholin-, Triäthanolamin-Salze.

Beispiel 1

2-Oxa-3-oxo-6-[3-(bicyclo[2,2,1]hept-5-en-2-yl)-3-oxo-prop-1-en-1-yl]-7-(p-phenylbenzoyloxy)-bicyclo[3,3,0]octan

Zu 66,7 Gew.-Teilen 2-(Bicyclo[2,2,1]hept-5-en-2-yl)-2-oxo-äthan-phosphonsäuredimethylester in 1300 Volumenteilen wasserfreiem Tetrahydrofuran läßt man unter Intergas 108,7 Volumenteile Butyllithium (2,2-molare Lösung in Hexan) bei 25°C einfließen. Man rührt 15 Min. und gibt darauf 79,7 Gew.-Teile (±)-2-Oxa-3-oxo-6-formyl-7-(p-phenylbenzoyloxy)-bicyclo[3,3,0]octan in 850 Volumenteilen wasserfreiem Dimethoxyäthan zu, rührt 1 Stunde bei 25°C, neutralisiert mit Essigsäure, dampft die Lösungsmittel im Vakuum ab, nimmt in Chloroform auf, wäscht mit gesättigter wäßriger Natriumhydrogencarbonat-Lösung und gesättigter wäßriger Natriumchlorid-Lösung, trocknet und engt im Vakuum ein. Der Rückstand wird mit 260 Volumenteilen Hexan und 260 Volumenteilen iso-Propanol versetzt. Man trennt den Niederschlag ab und kristallisiert aus 4300 Volumenteilen Äthanol um. Man erhält 76,0 Gew.-Teile 2-Oxo-3-oxo-6-[3-(bicyclo[2,2,1]hept-5-en-2-yl)-3-oxo-prop-1-en-1-yl]-7-(p-phenylbenzoyl-oxy)-bicyclo[3,3,0]octan (Ausbeute: 71 % der Theorie). Fp = 164-165°C.

Analyse: $C_{30}H_{28}O_5$ (Molekulargewicht 468)

| | | | |
|---|---|---|---|
| Ber.: | C 76,90 | H 6,02 | O 17,05 |
| Gef.: | 76,7 | 6,0 | 17,2 |

Beispiele 2-10

Man setzt 1 Mol eines Aldehyds der allgemeinen Formel (II) mit 1,05 bis 1,3 Mol eines Phosphonesters der allgemeinen Formel (III), der vorher mit 1,05 bis 1,15 Mol einer Base, gegebenenfalls Butyllithium versetzt wurde, in der in Beispiel 1 beschriebenen Weise um und erhält die in Tabelle 1 aufgeführten Reaktionsprodukte.

Tabelle 1: Hergestellte Verbindungen der allgemeinen Formel (IV)

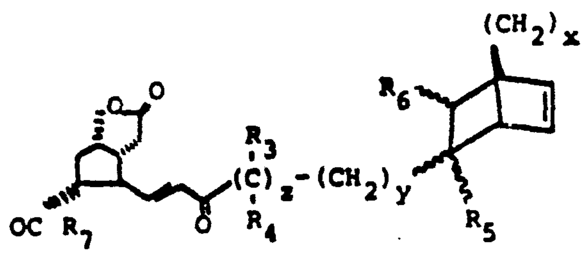

(IV)

| Beispiel Nr. | $R_3$ | $R_4$ | $R_5$ | $R_6$ | $R_7$ | x | y | z | Ausbeute in % der Theorie |
|---|---|---|---|---|---|---|---|---|---|
| 2 | H | $CH_3$ | H | H | 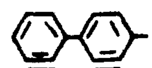 | 1 | 0 | 1 | 65 |
| 3 | $CH_3$ | $CH_3$ | H | H | 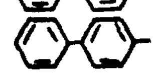 | 1 | 0 | 1 | 72 |
| 4 | H | H | H | H | 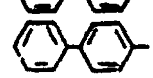 | 1 | 4 | 1 | 71 |
| 5 | - | - | H | $CH_3$ | 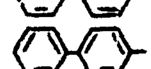 | 1 | 0 | 0 | 67 |
| 6 | - | - | $CH_3$ | H | 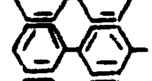 | 1 | 0 | 0 | 75 |
| 7 | - | - | H | H | 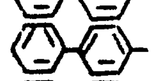 | 2 | 0 | 0 | 69 |
| 8 | $CH_3$ | H | H | H | 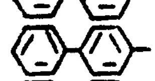 | 2 | 3 | 1 | 76 |
| 9 | - | - | H | H | 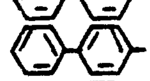 | 3 | 0 | 0 | 73 |
| 10 | - | - | H | $CH_3$ | 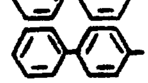 | 3 | 0 | 0 | 64 |

Beispiel 11

2-Oxa-3-oxo-6-[3-(bicyclo[2,2,1]hept-5-en-2-yl)-3-hydroxy-prop-1-en-1-yl]-7-(p-phenylbenzoyloxy)-bicyclo[3,3,0]octan

Zu 94.3 Gewichtsteilen wasserfreiem Zinkchlorid in 740 Volumenteilen wasserfreiem Dimethoxyäthan gibt man rasch bei 0°C 22,5 Gewichtsteile Natriumborhydrid, rührt 1 Stunde bei 0°C und 10 Stunden bei 20°C. Dazu läßt man bei -30°C 60,0 Gewichtsteile 2-Oxa-3-oxo-6-[3-(bicyclo[2,2,1]hept-5-en-2-yl)-3-oxo-prop-1-en-1-yl]-7-(p-phenylbenzoyloxy)-bicyclo[3,3,0]octan in 1050 Volumenteilen wasserfreiem Dimethoxyäthan fließen. Man rührt 5 Stunden bei -30°C, 4 Stunden bei -20°C und je 2 Stunden bei 0°C und 25°C. Bei 0°C läßt man darauf 130 Volumenteile Wasser einfließen, verdünnt mit 2500 Volumenteilen Essigester, saugt ab und dampft das Lösungsmittel im Vakuum ab. Der Rückstand wird in Essigester aufgenommen, mit gesättigter wäßriger Kochsalzlösung gewaschen und getrocknet. Den nach dem Abdampfen des Essigesters erhaltenen Rückstand chromatographiert man an Kieselgel mit Chloroform und Aceton (9:1) und erhält insgesamt 27,2 Gewichtsteile 2-Oxa-3-oxo-6-[3-(bicyclo[2,2,1]hept-5-en-2-yl)-3-hydroxy-prop-1-en-1-yl]-7-(p-phenylbenzoyloxy)-bicyclo[3,3,0]-octan (Ausbeute: 45% der Theorie) in Form der aufgetrennten Diastereomerenpaare ($R_f$ = 032, $R_f$ = 0,20). Das Verhältnis liegt bei etwa 1:1.

$^1$H-NMR ($CDCl_3$): $\delta$ = 5,47-5,83 (Multiplett, 2H), 5,20-5,47 (Multiplett, 1H) und 4,83 - 5,20 ppm (Multiplett, 1H).

Beispiele 12-20

Man setzt 1 Mol eines Ketons der allgemeinen Formel (IV) mit ca. 1-2,5 Mol, gegebenenfalls in situ dargestellten Zinkborhydrid in der in Beispiel 11 beschriebenen Weise um und erhält die in Tabelle 2 aufgeführten Reaktionsprodukte.

Tabelle 2 Hergestellte Verbindungen der allgemeinen Formel (V)

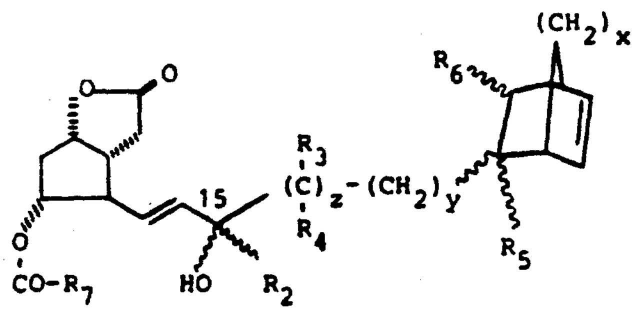

(V)

| Beispiel Nr. | $R_2$ | $R_3$ | $R_4$ | $R_5$ | $R_6$ | $R_7$ | x | y | z | Ausbeute % d.Th.* |
|---|---|---|---|---|---|---|---|---|---|---|
| 12 | H | H | $CH_3$ | H | H | 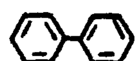 | 1 | 0 | 1 | 55 |
| 13 | H | $CH_3$ | $CH_3$ | H | H | 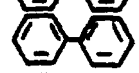 | 1 | 0 | 1 | 48 |
| 14 | H | H | H | H | H | 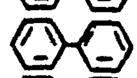 | 1 | 4 | 1 | 53 |
| 15 | H | - | - | H | $CH_3$ | 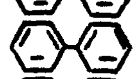 | 1 | 0 | 0 | 49 |
| 16 | H | - | - | $CH_3$ | H | 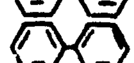 | 1 | 0 | 0 | 56 |
| 17 | H | - | - | H | H | 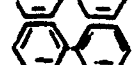 | 2 | 0 | 0 | 48 |
| 18 | H | $CH_3$ | H | H | H | 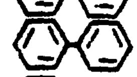 | 2 | 3 | 1 | 53 |
| 19 | H | - | - | H | H | 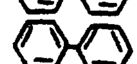 | 3 | 0 | 0 | 55 |
| 20 | H | - | - | H | $CH_3$ | 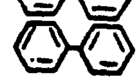 | 3 | 0 | 0 | 52 |

*) Gesamtausbeute der in 15α bzw. 15ß aufgetrennten Diaseteromeren

Beispiel 21

2-Oxa-3-oxo-6-[3-(bicyclo[2,2,1]hept-5-en-2-yl)-3-α-hydroxy-prop-1-en-1-yl]-7-(p-phenylbenzoyloxy)-bicyclo[3,3,0]octan

Zu 11,6 Gew.-Teilen 2-Oxa-3-oxo-6-[3-(bicyclo[2,2,1]hept-5-en-2-yl)-3-oxo-prop-1-en-1-yl]-7-(p-phenyl-benzoyloxy)-bicyclo[3,3,0]octan in 300 Volumenteilen wasserfreiem Dimethoxyäthan, 50 Volumenteilen wasserfreiem Tetrahydrofuran und 300 Volumenteilen wasserfreiem Diäthyläther läßt man unter Inertgas bei -102°C bis -104°C innerhalb von 90 Min. 150 Volumenteile Kalium-tri-sec.-butylborhydrid (0,5-molare Lösung in Tetrahydrofuran) einfließen und rührt 6 Stunden bei -104°C und 12 Stunden bei -78°C. Bei -70°C läßt man 150 Volumenteile Methanol / 75 Volumenteile 1n HCl einfließen. Man dampft ein, nimmt in 500 Volumenteilen Essigester auf, wäscht mit gesättigter wäßriger Natriumchloridlösung, trocknet und engt im Vakuum ein. Der Rückstand wird an Kieselgel mit Chloroform und Aceton (9:1) chromatographiert. Man erhält 4,6 Gew.-Teile 2-Oxa-3-oxo-6-[3-(bicyclo[2,2,1]hept-5-en-2-yl)-3α-hydroxy-prop-1-en-1-yl)-7-(p-phenylbenzoyloxy)-bicyclo[3,3,0]octan (Ausbeute 40 % der Theorie).

Ir: $\nu$ = 3410, 1775 und 1710 $cm^{-1}$

Beispiel 22

2-Oxa-3-oxo-6-[3-(bicyclo[2,2,1]hept-5-en-2-yl)-3α-hydroxy-prop-1-en-1-yl]-7-(p-phenylbenzoyloxy)-bicyclo[3,3,0]octan

Zu 4,7 Gew.-Teilen 2-Oxa-3-oxo-6-[3-(bicyclo[2,2,1]hept-5-en-2-yl)-3-oxo-prop-1-en-1-yl]-7-(p-phenylbenzoyloxy)-bicyclo[3,3,0]octan in 100 Volumenteilen wasserfreiem Toluol gibt man 10,2 Gew.-Teile Aluminiumtriisopropylat und erwärmt 6 Stunden unter Inertgas auf 80 bis 85°C. Man trägt unter Eiskühlung in 2n HCl aus, extrahiert mit Toluol, wäscht die Toluolphasen mit gesättigter wäßriger Natriumchloridlösung, trocknet, engt ein und chromatographiert den Rückstand an Kieselgel mit Chloroform und Aceton (9:1). Man erhält 1,4 g (30 % der Theorie) des in die Diastereomeren (siehe Beispiel 11) aufgetrennten 2-Oxa-3-oxo-6-[3-(bicyclo[2,2,1]hept-5-en-2-yl)-3-hydroxy-prop-1-en-1-yl]-7-(p-phenylbenzoyloxy)-bicyclo[3,3,0]octans.

IR: ν = 3410, 1775, 1710, 1280 und 1180 $cm^{-1}$.

Beispiel 23

2-Oxa-3-oxo-6-[8-(bicyclo[2,2,1]hept-5-en-2-yl)-3-methyl-3-hydroxy-oct-1-en-1-yl]7-(p-phenylbenzoyloxy)-bicyclo[3,3,0]octan

0,47 Gew.-Teile 2-Oxa-3-oxo-6-[8-(bicyclo[2,2,1]hept-5-en-2-yl)-3-oxo-oct-1-en-1-yl]-7-(p-phenylbenzoyloxy)-bicyclo[3,3,0]octan werden in 10 Volumenteilen wasserfreiem Tetra-

hydrofuran bei $-15^{O}C$ rasch unter Inertgas mit 0,73 Volumenteilen einer 2,7 molaren Lösung von Methylmagnesiumjodid in Diäthyläther versetzt und 1 Stunde bei $-25^{O}C$ gerührt. Bei $-25^{O}C$ läßt man 3 Volumenteile einer gesättigten wäßrigen Ammoniumchloridlösung einfließen. Man dampft die Lösungsmittel im Vakuum ein, nimmt den Rückstand in Diäthyläther auf, wäscht mit gesättigter wäßriger Natriumchloridlösung, trocknet und chromatographiert den nach dem Abdampfen des Äthers erhaltenen Rückstand an Kieselgel mit Methylenchlorid und Aceton (9:1). Man erhält 0,26 Gew.-Teile 2-Oxa-3-oxo-6-/3-(bicyclo/2,2,1/hept-5-en-2-yl)-3-methyl-3-hydroxy-oct-1-en-1-yl/-7-(p-Phenylbenzoyloxy)-bicyclo/3,3,0/octan (Ausbeute: 55 % der Theorie).

$^{1}H$-NMR ($CDCl_3$): $\delta$ = 1,27 (Singulett) ppm.

**Beispiel 24**

2-Oxa-3-oxo-6-/3-(bicyclo/2,2,1/hept-5-en-2-yl)-3α-hydroxy-prop-1-en-1-yl/-7-hydroxy-bicyclo/3,3,0/octan

39,4 Gew.-Teile 2-Oxa-3-oxo-6-/3-(bicyclo/2,2,1/hept-5-en-2-yl)-3α-hydroxy-prop-1-en-1-yl/-7-(p-phenylbenzoyloxy-)bicyclo /3,3,0/octan und 12,5 Gew.-Teile Kaliumcarbonat rührt man 90 Minuten in 350 Volumenteilen Methanol. Man neutralisiert mit 1n HCl, trennt den Niederschlag ab, verdampft das Lösungsmittel im Vakuum und extrahiert den Rückstand mit Essigester. Die Essigesterphase wird mit gesättigter wäßriger Natriumchloridlösung gewaschen und getrocknet. Der nach dem

Abdampfen des Essigesters verbleibende Rückstand wird an Kiesel mit 10 % Methanol enthaltenden Methylenchlorid chromatographiert. Man erhält 21,6 Gew.-Teile 2-Oxa-3-oxo-6-/3-(bicyclo/2,2,1/hept-5-en-2-yl)-3α-hydroxy-prop-1-en-1-yl/-7-hydroxy-bicyclo/3,3,0/octan (Ausbeute: 88 % der Theorie).

IR: $\nu$ = 3420 und 1765 $cm^{-1}$.

Beispiele 25-35

Man setzt 1 Mol eines p-Phenylbenzoesäureesters der allgemeinen Formel (V) in der in Beispiel 24 beschriebenen Weise mit 1-1,1 Mol Kaliumcarbonat um und erhält die in Tabelle 3 beschriebenen Reaktionsprodukte.

Tabelle 3: Hergestellte Verbindungen der allgemeinen Formel (VI)

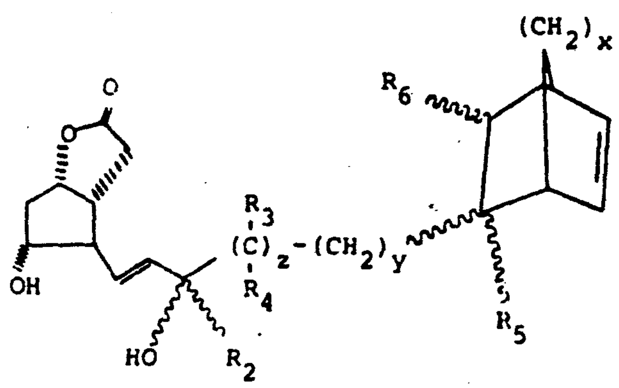

(VI)

| Beispiel Nr. | $R_2$ | $R_3$ | $R_4$ | $R_5$ | $R_6$ | x | y | z | Ausbeute in % der Theorie |
|---|---|---|---|---|---|---|---|---|---|
| 25 | H | H | $CH_3$ | H | H | 1 | O | 1 | 82 |
| 26 | H | $CH_3$ | $CH_3$ | H | H | 1 | O | 1 | 79 |
| 27 | H | H | H | H | H | 1 | 4 | 1 | 83 |
| 28 | $CH_3$ | H | H | H | H | 1 | 4 | 1 | 83 |
| 29 | H | - | - | H | $CH_3$ | 1 | O | O | 75 |
| 30 | H | - | - | $CH_3$ | H | 1 | O | O | 80 |
| 31 | H | - | - | H | H | 2 | O | O | 76 |
| 32 | H | $CH_3$ | H | H | H | 2 | 3 | 1 | 81 |
| 33 | H | - | - | H | H | 3 | O | O | 84 |
| 34 | H | - | - | H | $CH_3$ | 3 | O | O | 78 |

Beispiel 35

2-Oxa-3-oxo-6-[3-(bicyclo[2,2,1]hept-5-en-2-yl)-3 -tetrahydropyranyloxy-prop-1-en-1-yl]-7-tetrahydropyranyloxy-bicyclo[3,3,0]octan

Zu 27,0 Gew.-Teilen 2-Oxa-3-oxo-6-[3-(bicyclo[2,2,1]heot-5-en-2-yl)-3α-hydroxy-prop-1-en-1-yl]-7-hydroxy-bicyclo-[3,3,0]octan und 31,4 Gew.-Teilen Dihydropyran in 400 Volumenteilen Methylenchlorid läßt man 20 Volumenteile einer 2%igen p-Toluolsulfonsäure in Tetrahydrofuran einfließen. Man rührt 1 Stunde bei 25°C, gibt darauf 40 Volumenteile Pyridin und 1500 Volumenteile Essigester zu. Man wäscht mit gesättigter wäßriger Natriumhydrogencarbonatlösung und gesättigter wäßriger Natriumchloridlösung, trocknet, dampft die Lösungsmittel im Vakuum ein und chromatographiert den

Rückstand an Kieselgel mit Cyclohexan und Aceton (2:1). Man erhält 31,9 Gew.-Teile 2-Oxa-3-oxo-6-[3-(bicyclo[2,2,1]hept-5-en-2-yl)-3α-tetra-hydropyranyloxy-prop-1-en-1-yl]-7-tetrahydropyranyloxy-bicyclo[3,3,0]octan (Ausbeute: 75 % der Theorie).

IR: $\nu$ : 1772 $cm^{-1}$.

Beispiele 36-45

Man setzt 1 Mol eines Diols der allgemeinen Formel (VI) in der in Beispiel 35 beschriebenen Weise mit 1,5-5 Mol Dihydropyran um und erhält die in der Tabelle 4 beschriebenen Reaktionsprodukte.

Tabelle 4: Hergestellte Verbindungen der allgemeinen Formel (VII)

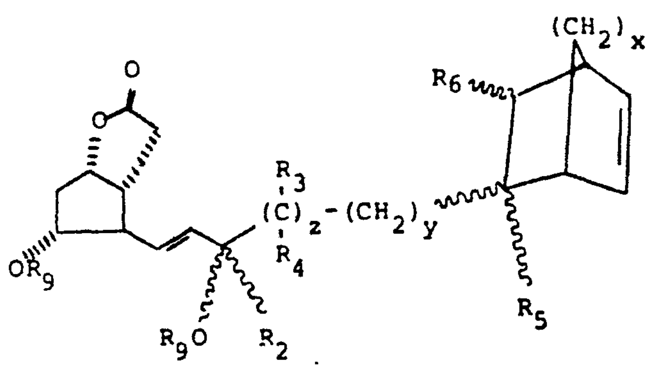

(VII)

| Beispiel Nr. | $R_2$ | $R_3$ | $R_4$ | $R_5$ | $R_6$ | $R_9$ | x | y | z | Ausbeute in % der Theorie |
|---|---|---|---|---|---|---|---|---|---|---|
| 36 | H | H | $CH_3$ | H | H | THP | 1 | 0 | 1 | 77 |
| 37 | H | $CH_3$ | $CH_3$ | H | H | THP | 1 | 0 | 1 | 80 |
| 38 | H | H | H | H | H | THP | 1 | 4 | 1 | 76 |
| 39 | $CH_3$ | H | H | H | H | THP | 1 | 4 | 1 | 79 |
| 40 | H | - | - | H | $CH_3$ | THP | 1 | 0 | 0 | 82 |
| 41 | H | - | - | $CH_3$ | H | THP | 1 | 0 | 0 | 84 |
| 42 | H | - | - | H | H | THP | 2 | 0 | 0 | 78 |
| 43 | H | $CH_3$ | H | H | H | THP | 2 | 3 | 1 | 79 |
| 44 | H | - | - | H | H | THP | 3 | 0 | 0 | 83 |
| 45 | H | - | - | H | $CH_3$ | THP | 3 | 0 | 0 | 84 |

THP ≙ 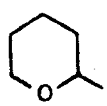

**Beispiel 46**

2-Oxa-3-oxo-6-[3-(bicyclo[2,2,2]oct-5-en-2-yl)-3α-(tert.-butyl-dimethyl-silyloxy)-prop-1-en-1-yl]-7-(tert.-dimethyl-silyloxy)-bicyclo[3,3,0]octan

4,56 Gew.-Teile [2-Oxa-3-oxo-6-[3-(bicyclo[2,2,2]oct-5-en-2-yl)-3α-hydroxy-prop-1-en-1-yl]-7-hydroxy-bicyclo[3,3,0]octan werden zusammen mit 6,12 Gew.-Teilen Imidazol, 6,0 Volumenteilen Dimethylformamid und 5,27 Gew.-Teilen tert-Butyl-dimethyl-silylchlorid 10 Stunden bei 37°C unter Inertgas gerührt. Man läßt 12 Stunden bei 25°C stehen, entfernt das Lösungsmittel bei 40°C im Vakuum, verdünnt den Abdampfrückstand mit Methylenchlorid, wäscht die organische Phase mit Wasser, trocknet sie über Natriumsulfat und entfernt das Lösungsmittel im Vakuum. Der Abdampfrückstand wird an Kieselgel mit Methylenchlorid chromatographiert. Man erhält 6,5 Gew.-Teile 2-Oxa-3-oxo-6-[3-(bicyclo[2,2,2]oct-5-en-2-yl)-3α-(tert.-butyl-dimethyl-silyloxy)-prop-1-en-1-yl]-7-(tert.-butyl-dimethyl-silyloxy)-bicyclo[3,3,0]octan (Ausbeute: 81 % der Theorie).

$^1$H-NMR ($CDCl_3$): δ = 4,70 - 5,05 (Multiplett, 1H), 0,85 (Singulett, 9H) und 0,83 ppm (Singulett, 9H).

Beispiel 47

2-Oxa-3-hydroxy-6-[3-(bicyclo[2,2,1]hept-5-en-2-yl)-3α-tetrahydropyranyloxy-pro-1-en-1-yl]-7-tetrahydropyranyloxybicyclo[3,3,0]octan

Zu 5,0 Gew.-Teilen 2-Oxa-3-oxo-6-[3-(bicyclo[2,2,1]hept-5-en-2-yl)-3α-tetrahydropyranyloxy-prop-1-en-1-yl]-7-tetrahydropyranyloxy-bicyclo[3,3,0]octan in 70 Volumenteilen wasserfreiem Toluol läßt man unter Inertgasatmosphäre 13 Volumenteile einer 70%igen Lösung von Natrium-bis-(2-methoxyäthoxy)-dihydridoaluminat in Benzol verdünnt mit 60 Volumenteilen wasserfreiem Toluol bei -70°C bis -78°C einfließen. Man rührt 6 Stunden in diesem Temperaturintervall. Bei -70°C läßt man darauf 43 Volumenteile Wasser und 43 Volumenteile Methanol einfließen, versetzt mit 130 Volumenteilen gesättigter wäßriger Natriumchloridlösung, extrahiert mit Essigester, wäscht mit gesättigter wäßriger Natriumchlorid-lösung, trocknet und dampft die Lösungsmittel im Vakuum ab. Man erhält 5,0 Gew.-Teile 2-Oxa-3-hydroxy-6-[3-(bicyclo[2,2,1]-hept-5-en-2-yl)-3α-tetrahydropyranyloxy-prop-1-en-1-yl]-7-tetra-hydropyranyloxy-bicyclo[3,3,0]octan. (Ausbeute: 100 % der Theorie).

IR: $\nu$ = 3380, 1080, 1022 und 980 $cm^{-1}$.

<u>Beispiele 48-57</u>

Man setzt 1 Mol eines Lactons der allgemeinen Formel (VII) in der in Beispiel 47 beschriebenen Weise mit 1-4,5 Mol Natrium-bis-(2-methoxyäthoxy)-di-hydrido-aluminat um und erhält die in Tabelle 5 beschriebenen Reaktionsprodukte.

<u>Tabelle 5:</u> Hergestellte Verbindungen der allgemeinen Formel (VIII)

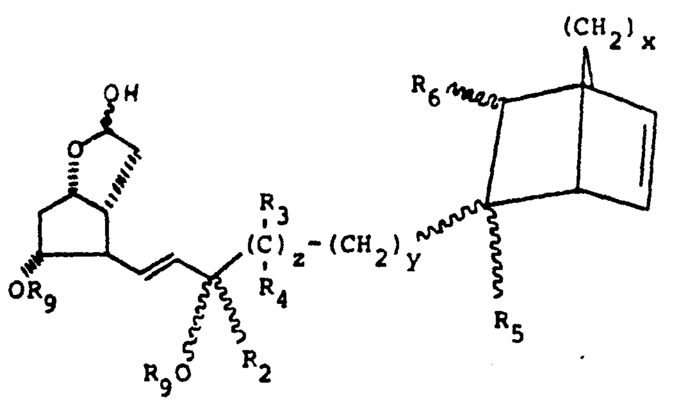

(VIII)

| Beispiel Nr. | $R_2$ | $R_3$ | $R_4$ | $R_5$ | $R_6$ | $R_9$ | x | y | z | Ausbeute in % der Theorie |
|---|---|---|---|---|---|---|---|---|---|---|
| 48 | H | H | $CH_3$ | H | H | THP | 1 | 0 | 1 | 98 |
| 49 | H | $CH_3$ | $CH_3$ | H | H | THP | 1 | 0 | 1 | 100 |
| 50 | H | H | H | H | H | THP | 1 | 4 | 1 | 99 |
| 51 | $CH_3$ | H | H | H | H | THP | 1 | 4 | 1 | 98 |
| 52 | H | - | - | H | $CH_3$ | THP | 1 | 0 | 0 | 100 |
| 53 | H | - | - | $CH_3$ | H | THP | 1 | 0 | 0 | 97 |
| 54 | H | - | - | H | H | THP | 2 | 0 | 0 | 100 |
| 55 | H | $CH_3$ | H | H | H | THP | 2 | 3 | 1 | 96 |
| 56 | H | - | - | H | H | THP | 3 | 0 | 0 | 98 |
| 57 | H | - | - | H | $CH_3$ | THP | 3 | 0 | 0 | 100 |

THP ≙ 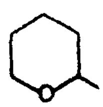

Beispiel 58

(5Z, 9α, 11α, 13E, 15α)-9-Hydroxy-11,15-di-tetrahydro-pyranyloxy-15-(bicyclo[2,2,1]hept-5-en-2-yl)-16,17,18,19,20-penta-nor-prosta-5,13-dien-1-säure

Unter Inertgas läßt man bei 5°C bis 10°C 13,6 Gew.-Teile Triphenyl-4-carboxy-butylphosphoniumbromid in 70 Volumenteilen wasserfreiem Dimethoxyäthan zu 11,5 Gew.-Teilen Natriumbis-(trimethylsilyl)-amid in 105 Volumenteilen

wasserfreiem Dimethoxyäthan einfließen und rührt 1 Stunde bei 25°C. Darauf läßt man bei 5°C bis 10°C 4,8 Gew.-Teile 2-Oxa-3-hydroxy-6-[3-(bicyclo[2,2,1]hept-5-en-2-yl)-3α-tetrahydropyranyloxy-prop-1-en-1-yl]-7-tetrahydropyranyloxy-bicyclo[3,3,0]octan in 70 Volumenteilen wasserfreiem Dimethoxyäthan einfließen und rührt noch 3 Stunden bei 25°C. Man gibt 3 Volumenteile Wasser zu, dampft das Lösungsmittel im Vakuum ab, verteilt den Rückstand zwischen 160 Volumenteilen Wasser und 4x 100 Volumenteilen Diäthyläther. Die wäßrige Phase wird mit Oxalsäure unter Kühlung auf pH 4 eingestellt und mit Pentan und Diäthyläther (1:1) extrahiert. Man trocknet und erhält nach dem Abdampfen des Lösungsmittels 4,4 Gew.-Teile (5Z, 9α, 11α, 13E, 15α)-9-Hydroxy-11,15-di-tetrahydropyranyloxy-15-(bicyclo[2,2,1]hept-5-en-2-yl)-16,17,18,19,20-penta-nor-prosta-5,13-dien-1-säure (Ausbeute 78 % der Theorie).

IR: ν : 1720 und 980 $cm^{-1}$.

**Beispiele 59-68**

Man setzt 1 Mol eines Lactols der allgemeinen Formel (VIII) in der in Beispiel 58 beschriebenen Weise mit 1,3 bis 3,0 Mol Phosphoniumsalz der allgemeinen Formel (IX) um und erhält die in der Tabelle 6 beschriebenen Reaktionsprodukte.

Tabelle 6: Hergestellte Verbindungen der allgemeinen Formel (X)

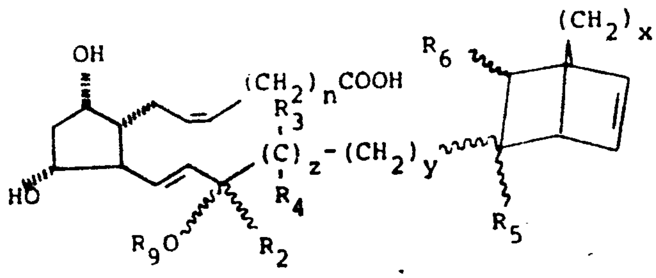

(X)

| Beispiel Nr. | $R_2$ | $R_3$ | $R_4$ | $R_5$ | $R_6$ | $R_9$ | x | y | z | n | Ausbeute in % d.Theorie |
|---|---|---|---|---|---|---|---|---|---|---|---|
| 59 | H | H | $CH_3$ | H | H | THP | 1 | 0 | 1 | 3 | 74 |
| 60 | H | $CH_3$ | $CH_3$ | H | H | THP | 1 | 0 | 1 | 3 | 60 |
| 61 | H | H | H | H | H | THP | 1 | 4 | 1 | 3 | 73 |
| 62 | $CH_3$ | H | H | H | H | THP | 1 | 4 | 1 | 3 | 77 |
| 63 | H | - | - | H | $CH_3$ | THP | 1 | 0 | 0 | 3 | 80 |
| 64 | H | - | - | $CH_3$ | H | THP | 1 | 0 | 0 | 3 | 80 |
| 65 | H | - | - | H | H | THP | 2 | 0 | 0 | 3 | 76 |
| 66 | H | $CH_3$ | H | H | H | THP | 2 | 3 | 1 | 3 | 77 |
| 67 | H | - | - | H | H | THP | 3 | 0 | 0 | 3 | 81 |
| 68 | H | - | - | H | $CH_3$ | THP | 3 | 0 | 0 | 3 | 73 |

THP ≙ 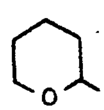

Beispiel 69

(5Z, 9α, 11α, 13E, 15α)-9-Hydroxy-11-15-di-tetrahydropyranyl-oxy-15-methyl-20-(bicyclo/2,2,1/hept-5-en-2-yl)-prosta-5,13-dien-1-säure

3,06 Gew.-Teile Natriumhydrid gibt man zu 55 Volumenteilen wasserfreiem Dimethylsulfoxid unter Inertgas und erwärmt 2 Stunden auf 60 bis 70°C. Dazu läßt man bei 15°C bis 16°C 28,1 Gew.-Teile Triphenyl-4-carboxy-butylphosphoniumbromid in120 Volumenteilen wasserfreiem Dimethylsulfoxid einfließen. Darauf läßt man 12,43 Gew.-Teile 2-Oxa-3-hydroxy-6-/8-(bicyclo/2,2,1/hept-5-en-2-yl)-3α-tetrahydropyranyloxy-3ß-methyl-oct-en-1-yl/-7-tetrahydropyranyloxy-bicyclo/3,3,0/octan in 100 Volumenteilen wasserfreiem Dimethylsulfoxid bei 15°C bis 16°C einfließen und rührt noch 2 Stunden bei 25°C. Nach Zugabe von 4,5 Volumenteilen Wasser wird das Lösungsmittel im Vakuum abdestilliert und der Rückstand zwischen 200 Volumenteilen Wasser und 4 x 260 Volumenteilen Diäthyläther verteilt. Die wäßrige Phase wird mit Oxalsäure auf pH 4 unter Kühlung eingestellt und mit Pentan und Diäthyläther (1:1) extrahiert. Man trocknet und erhält nach dem Abdampfen des Lösungsmittels 8,1 Gew.-Teile (5Z, 9α, 11α, 13E, 15α)-9-Hydroxy-11,15-di-tetrahydropyranyloxy-15-methyl-20(bicyclo/2,2,1/hept-5-en-2-yl)-prosta-5,13-dien-1-säure (Ausbeute: 56 % der Theorie).

IR: ν : 1710 $cm^{-1}$

Beispiel 70

(5Z,9α, 11α, 13E, 15α)-9,11,15-Trihydroxy-15-(bicyclo /2,2,1/hept-5-en-2-yl)-16,17,18,19,20-penta-nor-prosta-5,13-dien-1-säure

4,0 Gew.-Teile (5Z, 9α, 11α, 13E, 15α)-9-Hydroxy-11,15-di-tetrahydropyranyloxy-15-(bicyclo[2,2,1]hept-5-en-2-yl)-16,17,18,19,20-penta-nor-5,13-dien-1-säure beläßt man 4 Tage in 30 Volumenteilen Methanol und 20 Volumenteilen Essigsäure bei $20^{\circ}C$. Man dampft die Lösungsmittel im Vakuum ab, chromatographiert den Rückstand an Kieselgel mit Chloroform, Essigsäure und Tetrahydrofuran (10:3:5) und erhält 2,1 Gew.-Teile (5Z, 9α, 11α, 13E, 15α)-9,11,15-Trihydroxy-15-(bicyclo[2,2,1]hept-5-en-2-yl)-16,17,18,19,20-penta-nor-prosta-5,13-dien-1-säure (Ausbeute: 76 % der Theorie).

IR: $\nu$ = 3360, 1715 und 975 $cm^{-1}$.

$^{1}H$-NMR ($CDCl_3$): $\delta$ = 5,91-6,33 (Multiplett, 2H) und 5,11-5,83 ppm (Multiplett, 4H).

Wahlweise läßt sich auch die Diastereomerentrennung (15α,15β), die in Beispiel 11 auf der Stufe des Allylalkohols (V) beschrieben wurde, durch Chromatographie der $C_{15}$-Diastereomerenpaare von (5Z, 9α, 11α, 13E, 15α/β)-9,11,15-Trihydroxy-15-(bicyclo[2,2,1]-hept-5-en-2-yl)-16,17,18,19,20-penta-nor-prosta-5,13-dien-1-säure an Kieselgel beispielsweise mit dem Lösungsmittelgemisch Essigsäure, Chloroform und Methanol gut durchführen.

Das gilt auch für die in Tabelle 7 aufgeführten Verbindungen.

Beispiele 71-80

Man setzt 1 Mol einer Säure der allgemeinen Formel (X) in der in Beispiel 70 beschriebenen Weise mit Essigsäure, Methanol um und erhält die in Tabelle 7 aufgeführten Reaktionsprodukte.

Tabelle 7: Hergestellte Verbindungen der allgemeinen Formel (Ia)

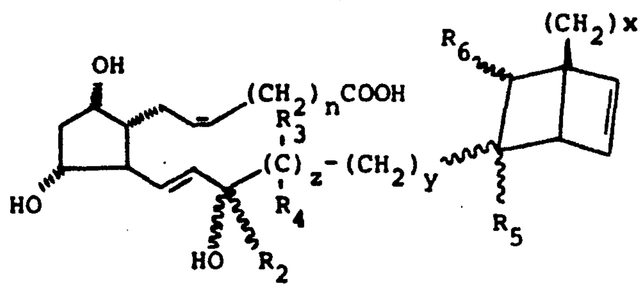

(Ia)

| Beispiel Nr. | $R_2$ | $R_3$ | $R_4$ | $R_5$ | $R_6$ | x | y | z | n | Ausbeute in % der Theorie |
|---|---|---|---|---|---|---|---|---|---|---|
| 71 | H | H | $CH_3$ | H | H | 1 | 0 | 1 | 3 | 70 |
| 72 | H | $CH_3$ | $CH_3$ | H | H | 1 | 0 | 1 | 3 | 65 |
| 73 | H | H | H | H | H | 1 | 4 | 1 | 3 | 67 |
| 74 | $CH_3$ | H | H | H | H | 1 | 4 | 1 | 3 | 73 |
| 75 | H | - | - | H | $CH_3$ | 1 | 0 | 0 | 3 | 64 |
| 76 | H | - | - | $CH_3$ | H | 1 | 0 | 0 | 3 | 69 |
| 77 | H | - | - | H | H | 2 | 0 | 0 | 3 | 72 |
| 78 | H | $CH_3$ | H | H | H | 2 | 3 | 1 | 3 | 63 |
| 79 | H | - | - | H | $CH_3$ | 3 | 0 | 0 | 3 | 66 |
| 80 | H | - | - | H | $CH_3$ | 3 | 0 | 0 | 3 | 70 |

Beispiel 81

(5Z, 11α, 13E, 15α)-9-Oxo-11,15-di-tetrahydropyranyloxy-15-(bicyclo[2,2,1]5-en-2-yl)-16,17,18,19,20-penta-nor-prosta-5,13-dien-1-säure

Zu 14,3 Gew.-Teilen (5Z, 9α, 11α, 13E, 15α)-9-Hydroxy-11,15-di-tetrahydropyranyloxy-15-(bicyclo[2,2,1]hept-5-en-2-yl)-16,17,18,19,20-penta-nor-prosta-5,13-dien-1-säure in 650 Volumenteilen Aceton läßt man bei -20°C, 36,2 Volumenteile einer schwefelsauren 2,67 molaren Chromtrioxidlösung in Wasser einfließen und rührt 3 Stunden bei -20°C. Bei -20°C läßt man darauf 100 Volumenteile 2-Propanol einfließen und stellt nach Zugabe von 34 Volumenteilen Wasser den pH auf 4,8 durch Versetzen mit Natriumhydrogencarbonat ein. Man filtriert ab, dampft das Lösungsmittel im Vakuum ein, nimmt in Essigester auf, trocknet und erhält nach dem Abdampfen des Lösungsmittels 12,9 Gew.-Teile (5Z, 11 , 13E, 15 )-9-Oxo-11,15-di-tetrahydropyranyloxy-15-(bicyclo[2,2,1]hept-5-en-2-yl)-16,17,18,19,20-penta-nor-prosta-5,13-dien-1-säure (Rohausbeute: 90 % der Theorie).

$^1$H-NMR ($CDCl_3$): $\delta$ = 5,96-6,33 (Multiplett, 2H), 5,22-5,76 (Multiplett, 4H) und 4,55-4,96 ppm (Multiplett, 2H)

Beispiele 82-91

Man setzt 1 Mol einer Säure der allgemeinen Formel (X) in der in Beispiel 81 beschriebenen Weise mit 2 bis 44 Mol Chromtrioxid um und erhält die in Tabelle 8 beschriebenen Reaktionsprodukte.

Tabelle 8: Hergestellte Verbindungen der allgemeinen Formel (XI)

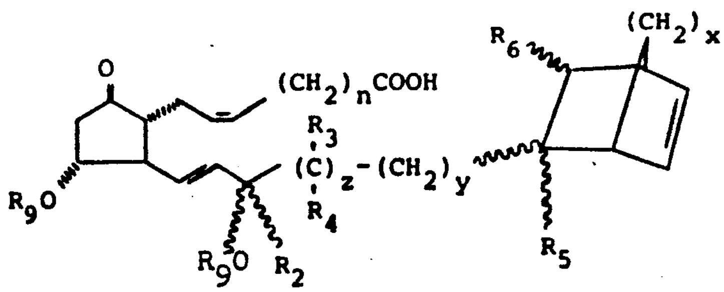

(XI)

| Beispiel Nr. | $R_2$ | $R_3$ | $R_4$ | $R_5$ | $R_6$ | $R_9$ | x | y | z | n | Ausbeute in % der Theorie* |
|---|---|---|---|---|---|---|---|---|---|---|---|
| 82 | H | H | $CH_3$ | H | H | THP | 1 | O | 1 | 3 | 87 |
| 83 | H | $CH_3$ | $CH_3$ | H | H | THP | 1 | O | 1 | 3 | 90 |
| 84 | H | H | H | H | H | THP | 1 | 4 | 1 | 3 | 82 |
| 85 | $CH_3$ | H | H | H | H | THP | 1 | 4 | 1 | 3 | 85 |
| 86 | H | - | - | H | $CH_3$ | THP | 1 | O | O | 3 | 91 |
| 87 | H | - | - | $CH_3$ | H | THP | 1 | O | O | 3 | 94 |
| 88 | H | - | - | H | H | THP | 2 | O | O | 3 | 87 |
| 89 | H | $CH_3$ | H | H | H | THP | 2 | 3 | 1 | 3 | 85 |
| 90 | H | - | - | H | H | THP | 3 | O | O | 3 | 87 |
| 91 | H | - | - | H | $CH_3$ | THP | 3 | O | O | 3 | 90 |

*) Rohausbeute

THP ≙ 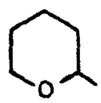

Beispiel 92

(5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säure

11,8 Gew.-Teile (5Z, 11α, 13E, 15α)-9-Oxo-11,15-di-tetrahydropyranyloxy-15(bicyclo[2,2,1]hept-5-en-2-yl)-16,17,18,19,20-penta-nor-prosta-5,13-dien-1-säure erwärmt man 4 Stunden in 75 Volumenteilen Essigsäure, Wasser, Tetrahydrofuran (2:1:0,3) auf 42°C. Man versetzt mit 75 Volumenteilen Wasser und destilliert die Lösungsmittel im Vakuum schonend ab. Der Rückstand liefert nach der Chromatographie an Kieselgel mit Chloroform, Tetrahydrofuran und Essigsäure (10:5:3) 4,1 Gew.-Teile (5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säure (Ausbeute: 50 % der Theorie).

IR: ν : 1735 und 1714 $cm^{-1}$

$^1H$-NMR ($CDCl_3$): δ: 5,91-6,33 (Multiplett,2H), 5,11-5,83 ppm (Multiplett,2H ).

Die Diastereomerentrennung (15α, 15ß) läßt sich auch statt auf der Stufe des Allylalkohols (V) (siehe Beispiel 11) durch Chromatographie der $C_{15}$-Diastereomerenpaare von (5Z, 11α, 13E, 15α/15ß)-9-Oxo-11,15-dihydroxy-15-(bicyclo/2,2,1/hept-5-en-2-yl)-16,17,18,19,20-penta-nor-prosta-5,13-dien-1-säure an Kieselgel mit beispielsweise obigem Gemisch durchführen.

Dies gilt auch für die in der Tabelle 9 aufgeführten Verbindungen.

## Beispiele 93-102

Man setzt 1 Mol einer Säure der allgemeinen Formel (XI) in der in Beispiel 92 beschriebenen Weise mit Essigsäure, Wasser und Tetrahydrofuran um und erhält die in Tabelle 9 aufgeführten Reaktionsprodukte.

<u>Tabelle 9:</u> Hergestellte Verbindungen der allgemeinen Formel (Ib)

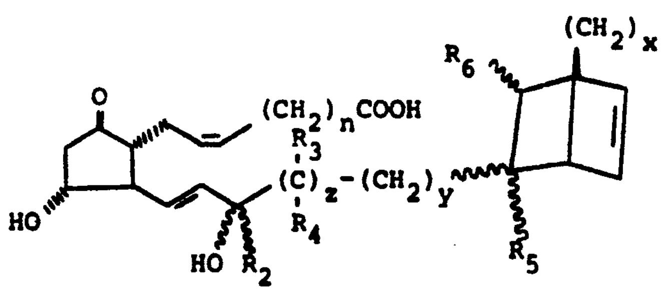

(Ib)

| Beispiel Nr. | $R_2$ | $R_3$ | $R_4$ | $R_5$ | $R_6$ | x | y | z | n | Ausbeute in % der Theorie |
|---|---|---|---|---|---|---|---|---|---|---|
| 93 | H | H | $CH_3$ | H | H | 1 | 0 | 1 | 3 | 45 |
| 94 | H | $CH_3$ | $CH_3$ | H | H | 1 | 0 | 1 | 3 | 48 |
| 95 | H | H | H | H | H | 1 | 4 | 1 | 3 | 52 |
| 96 | $CH_3$ | H | H | H | H | 1 | 4 | 1 | 3 | 49 |
| 97 | H | - | - | H | $CH_3$ | 1 | 0 | 0 | 3 | 46 |
| 98 | H | - | - | $CH_3$ | H | 1 | 0 | 0 | 3 | 51 |
| 99 | H | - | - | H | H | 2 | 0 | 0 | 3 | 43 |
| 100 | H | $CH_3$ | H | H | H | 2 | 3 | 1 | 3 | 48 |
| 101 | H | - | - | H | H | 3 | 0 | 0 | 3 | 49 |
| 102 | H | - | - | H | $CH_3$ | 3 | 0 | 0 | 3 | 52 |

**Beispiel 103**

(5Z, 13E, 15α)-9-Oxo-15-hydroxy-15-(bicyclo[2,2,1]hept-5-en-2-yl)-16,17,18,19,20-penta-nor-prosta-5,10,13-trien-1-säure

2,0 Gew.-Teile (5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säure in 10 Volumenteilen Tetrahydrofuran säuert man mit 1n HCl auf pH 2 an und beläßt den Ansatz 4 Tage bei 25°C. Mit gesättigter wäßriger Natriumhydrogencarbonatlösung stellt man auf pH 4 ein, engt im Vakuum ein, nimmt in wenig Essigester auf, trocknet, filtriert und chromatographiert an Kieselgel mit Methylenchlorid, Essigsäure und Tetrahydrofuran (10:3:5). Man erhält 0,9 Gew.-Teile (5Z, 13E, 15α)-9-Oxo-15-hydroxy-15-(bicyclo[2,2,1]hept-5-en-2-yl)-16,

17,18,19,20-penta-nor-prosta-5,10,13,trien-1-säure (Ausbeute: 41 % der Theorie).

Ir: ν: 1728 und 1711 $cm^{-1}$.

$^1H$-NMR ($CDCl_3$): δ =7,51 (Dublett 1/2 H), 7,59 (Dublett,1/2H) 6,02-6,28 (Multiplett, 2,5 H), 5,50-5,78 (Multiplett, 2H) und 5,22-5,50 ppm (Multiplett, 2H).

Beispiele 104-113

Man setzt 1 Mol einer Säure der allgemeinen Formel (Ib) mit beispielsweise Salzsäure in der in Beispiel 103 beschriebenen Weise um und erhält die in der Tabelle 10 aufgeführten Reaktionsprodukte.

Tabelle 10: Hergestellte Verbindungen der allgemeinen Formel (Ic)

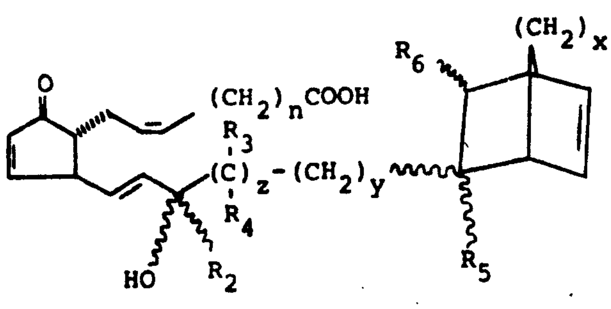

(Ic)

| Beispiel Nr. | $R_2$ | $R_3$ | $R_4$ | $R_5$ | $R_6$ | x | y | z | n | Ausbeute in % der Theorie |
|---|---|---|---|---|---|---|---|---|---|---|
| 104 | H | H | $CH_3$ | H | H | 1 | 0 | 1 | 3 | 46 |
| 105 | H | $CH_3$ | $CH_3$ | H | H | 1 | 0 | 1 | 3 | 46 |
| 106 | H | H | H | H | H | 1 | 4 | 1 | 3 | 39 |
| 107 | $CH_3$ | H | H | H | H | 1 | 4 | 1 | 3 | 38 |
| 108 | H | - | - | H | $CH_3$ | 1 | 0 | 0 | 3 | 42 |
| 109 | H | - | - | $CH_3$ | H | 1 | 0 | 0 | 3 | 43 |
| 110 | H | - | - | H | H | 2 | 0 | 0 | 3 | 40 |
| 111 | H | $CH_3$ | H | H | H | 2 | 3 | 1 | 3 | 35 |
| 112 | H | - | - | H | H | 3 | 0 | 0 | 3 | 42 |
| 113 | H | - | - | H | $CH_3$ | 3 | 0 | 0 | 3 | 39 |

## Beispiel 114

(5Z, 13E, 15α)-9-Oxo-15-hydroxy-15-(bicyclo[2,2,1]hept-5-en-2-yl)-16,17,18,19,20-penta-nor-prosta-5,10,13-trien-1-säure

Zu 1,3 Gew.-Teilen (5Z, 11α, 13E, 15α)-9-Oxo-11,15-di-tetrahydro-pyranyloxy-15-(bicyclo[2,2,1]-hept-5-en-2-yl)-16,17,18,19,20-penta-nor-prosta-5,13-dien-1-säure in 15 Volumenteilen Tetrahydrofuran gibt man 0,3 Volumenteile in wäßrige Salzsäure. Man beläßt 4 Tabe bei 20°C, stellt den pH darauf mit gesättigter wäßriger Natriumhydrogencarbonatlösung auf pH 4 ein. Nach dem Abdampfen des Lösungsmittels chromatographiert man den Rückstand an Kieselgel mit Methylenchlorid, Essigsäure und Tetrahydrofuran (10:3:5) und erhält 0,3 Gew.-Teile

(5Z, 13E, 15α)-9-Oxo-15-hydroxy-15-(bicyclo[2,2,1]hept-5-en-2-yl)-16,17,18,19,20-penta-nor-prosta-5,10,13-trien-1-säure (Ausbeute: 35 % der Theorie).

IR: ν : 1716 und 1730 $cm^{-1}$.

Beispiel 115

(5Z, 13E, 15α)-9-Oxo-15-hydroxy-15-(bicyclo[2,2,1]hept-5-en-2-yl)-16,17,18,19,20-penta-nor-prosta-5,8,13-trien-1-säure

Zu 0,5 Gew.-Teilen (5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säure in 3 Volumenteilen Methanol gibt man pulverisiertes Kaliumhydroxid bis zum pH 10. Man rührt 3 Stunden bei 25°C, säuert mit 10 %iger Salzsäure auf pH 4 an, engt im Vakuum ein, nimmt in Essigester auf, filtriert, trocknet und dampft dem Essigester im Vakuum ab. Man chromatographiert an Kieselgel mit Chloroform, Tetrahydrofuran und Essigsäure (20:2:1) und erhält 0,35 Gew.-Teile (5Z, 13E, 15α)-9-Oxo-15-hydroxy-15-(bicyclo[2,2,1]hept-5-en-2-yl)-16,17,18,19,20-penta-nor-prosta-5,8,13-trien-1-säure (Ausbeute: 74 % der Theorie).

IR: ν: 2260, 1735 und 1710 $cm^{-1}$.

Beispiele 116-117

Man versetzt 1 Mol einer Säure der allgemeinen Formel (Ib) in der in Beispiel 115 beschriebenen Weise bei einem pH von 9-11 um und erhält die in der Tabelle 11 aufgeführten Reaktionsprodukte.

Tabelle 11: Hergestellte Verbindungen der allgemeinen Formel (Id)

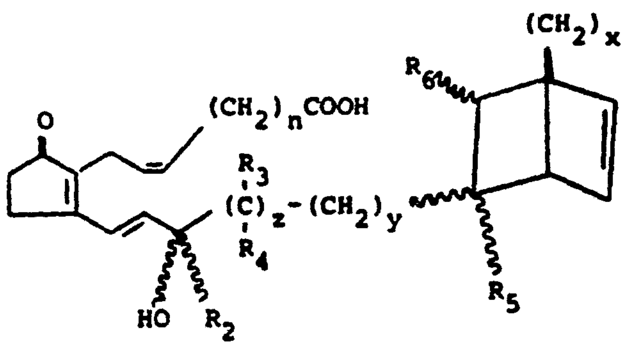

(Id)

| Beispiel Nr. | $R_2$ | $R_3$ | $R_4$ | $R_5$ | $R_6$ | x | y | z | n | Ausbeute in % der Theorie |
|---|---|---|---|---|---|---|---|---|---|---|
| 116 | H | H | H | H | H | 1 | 4 | 1 | 3 | 80 |
| 117 | H | $CH_3$ | H | H | H | 2 | 3 | 1 | 3 | 79 |

Beispiel 118

(5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säuremethylester

Zu 3,7 Gew.-Teilen (5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säure läßt man in 50 Volumenteilen Methanol und 5 Volumenteilen Wasser 10 Volumenteile einer 1,3-molaren ätherischen Diazomethanlösung einfließen. Man rührt 1 Stunde bei 20°C, zersetzt überschüssiges Diazomethan mit Essigsäure, dampft die Lösungsmittel ab, filtriert über Kieselgel mit Essigester und erhält nach dem Eindampfen des Essigesters 3,6 Gew.-Teile (5Z, 11α, 13E, 15α)-9-Oxo-11,15-dihydroxy-15-(bicyclo[2,2,1]hept-5-en-2-yl)-16,17,18,19,20-penta-nor-prosta-5,13-dien-1-säuremethylester (Ausbeute: 93 % der Theorie).

IR: $\nu$ 1730 $cm^{-1}$.

$^{1}H$-NMR ($CDCl_3$): $\delta$ = 3,66 ppm (Singulett, 3H).

0002215



Patentansprüche

1. Bicycloalkenyl-Prostaglandine der allgemeinen Formel (I)

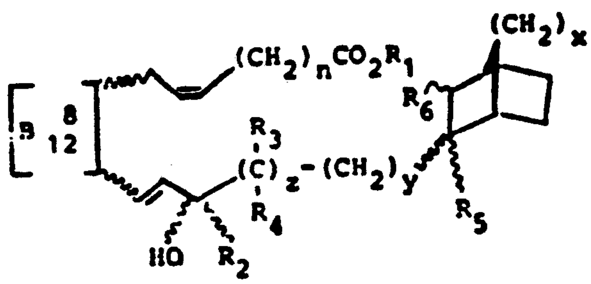

(I)

in welcher

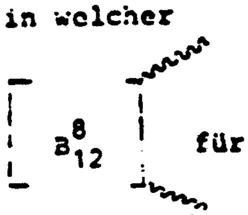

für die Teilstrukturen

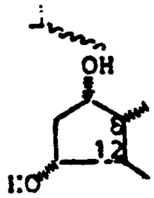 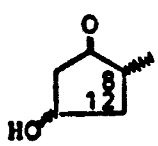 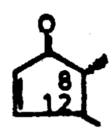 oder 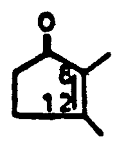

(Ia) (Ib) (Ic) (Id)

steht,

$R_1$ für Wasserstoff oder einen gegebenenfalls substituierten Alkylrest steht,

$R_2$ für Wasserstoff oder einen Alkylrest steht

$R_3$ und $R_4$ gleich oder verschieden sind und für Wasserstoff oder einen gegebenenfalls substituierten Alkylrest oder Aralkylrest stehen, wobei einer der Substituenten immer Wasserstoff ist, wenn der andere für Aralkyl steht,

$R_5$ und $R_6$ gleich oder verschieden sind und für Wasserstoff oder einen gegebenenfalls substituierten Alkylrest oder Aralkylrest stehen,

z für die Zahlen 0 bis 2 steht

y für die Zahlen 0 bis 6 steht,

x für die Zahlen 1 bis 4 steht,

n für die Zahlen 2 bis 6 steht und

falls $R_1$ Wasserstoff bedeutet, auch deren physiologisch verträgliche Salze.

2. Bicycloalkenyl-prostaglandine gemäß Anspruch 1 wobei

$R_1$ für Wasserstoff oder Alkyl mit 1 bis 6 Kohlenstoffatomen steht,

$R_2$ für Wasserstoff oder Alkyl mit 1 bis 4, insbesondere 1 oder 2 Kohlenstoffatomen steht,

$R_3$ und $R_4$ gleich oder verschieden sind und jeweils für Wasserstoff, Benzyl, oder Alkyl mit 1 bis 4 insbesondere 1 oder 2 Kohlenstoffatomen stehen, wobei einer der

Substituenten immer Wasserstoff ist wenn der andere für Benzyl steht,

$R_5$ und $R_6$ gleich oder verschieden sind und für Wasserstoff, Benzyl oder einen Alkylrest mit 1 bis 4 insbesondere 1 oder 2 Kohlenstoffatomen stehen,

z für 0,1 oder 2 steht,

y für eine Zahl von 0 bis 5 steht,

x für eine Zahl von 1 bis 3 steht und

n für eine Zahl von 2 bis 5 steht,

sowie deren physiologisch verträgliche Salze für den Fall, daß $R_1$ Wasserstoff bedeutet.

3. Verfahren zur Herstellung von Bicycloalkenyl-prostaglandinen gemäß Anspruch 1, dadurch gekennzeichnet, daß man gemäß dem Reaktionsschema (I) einen Aldehyd der allgemeinen Formel (II)

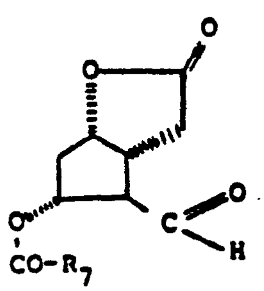

(II)

in welcher

$R_7$ für einen gegebenenfalls substituierten Alkyl- oder Arylrest steht, mit einen Phosphonester der allgemeinen Formel (III)

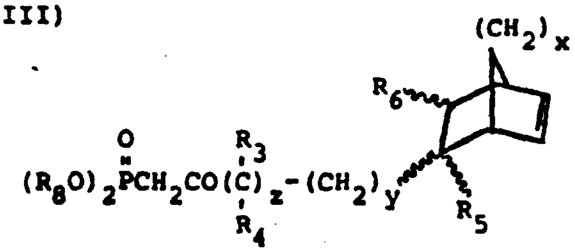

(III)

in welcher

$R_8$ für einen gegebenenfalls substituierten Alkylrest steht,

$R_3, R_4, R_5, R_6$, x, y und z die oben angegebene Bedeutung haben,

zu einen α,ß-ungesättigten Keton der Formel (IV)

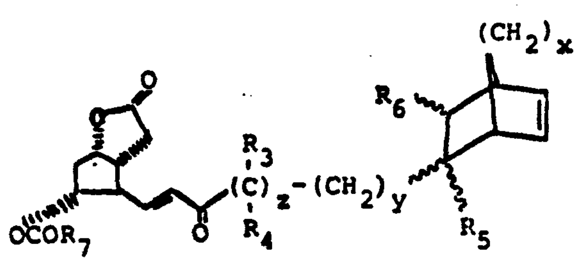

(IV)

in welcher

$R_3, R_4, R_5, R_6, R_7$, x, y und z die oben angegebene Bedeutung haben,

in Anwesenheit einer Base umsetzt und anschließend das α,ß-un-

gesättigte Keton (IV) mit einem komplexen Metallborhydrid, mit einem Aluminiumalkoholat oder gegebenenfalls mit einer metallorganischen Alkylverbindung zu einem Allylalkohol der allgemeinen Formel (V)

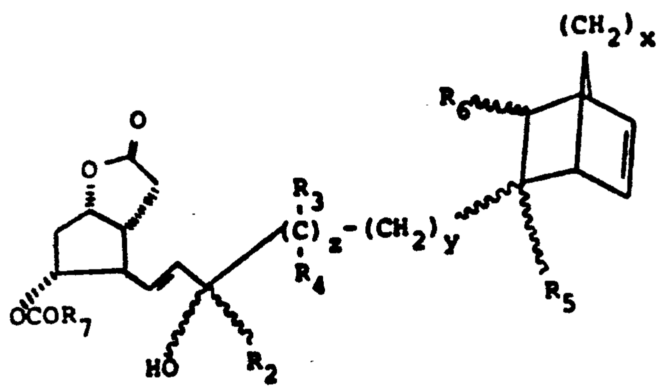

(V)

in welcher

$R_2, R_3, R_4, R_5, R_6, R_7$, x, y und z die oben aufgeführte Bedeutung haben

reduziert, diesen durch Abspaltung der Gruppe $COR_7$ in ein Diol (VI) überführt, gegebenenfalls Schutzgruppen einführt und das so erhaltene Lacton der allgemeinen Formel (VII)

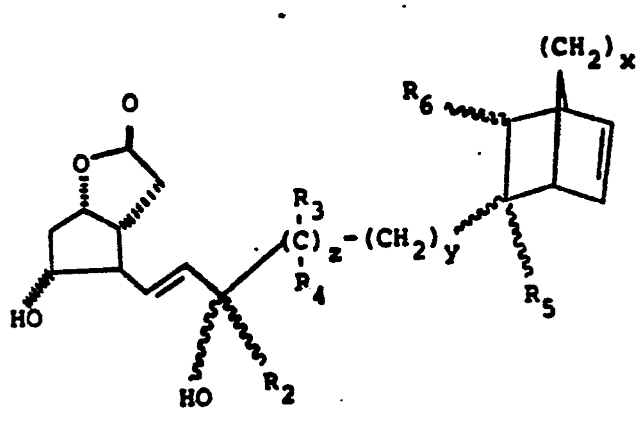

(VI)

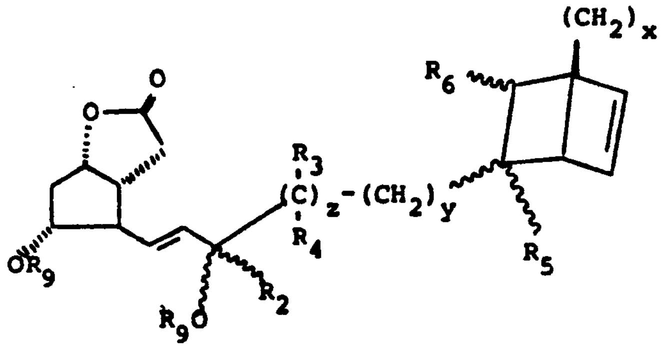

(VII)

in welcher

$R_9$ für einen Organosilyl- oder Ätherrest steht,

$R_2, R_3, R_4, R_5, R_6, x, y$ und $z$ die obige Bedeutung haben

zu einem Lactol der allgemeinen Formel (VIII)

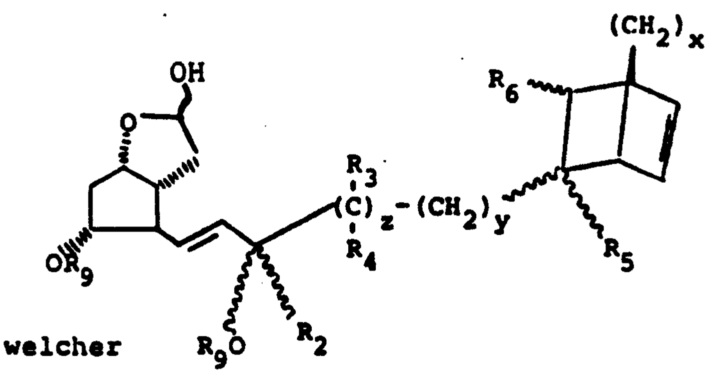

(VIII)

in welcher

$R_2, R_3, R_4, R_5, R_6, R_9, x, y$ und $z$ die obige Bedeutung haben

reduziert und das so erhaltene Lactol mit einem Phosphoniumsalz der allgemeinen Formel (IX)

$$[(R_{10})_3{}^{\oplus}PCH_2(CH_2)_nCO_2H]\ X^{\ominus} \qquad (IX)$$

in welcher

$R_{10}$ für einen Arylrest,

X für ein Halogen und

n für eine Zahl zwischen 2 und 6 stehen,

in Anwesenheit einer Base zu einer Säure der allgemeinen Formel (X)

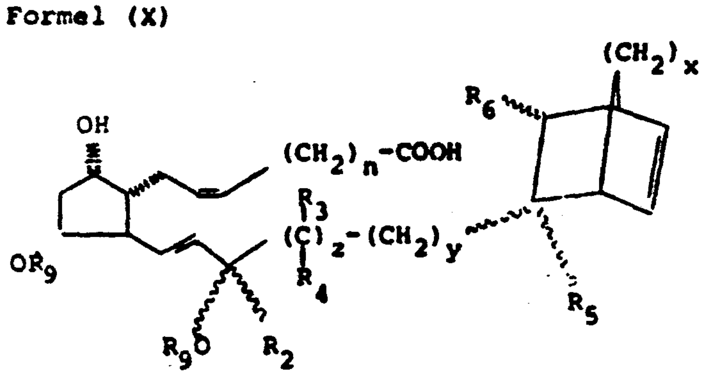

(X)

in welcher

$R_2, R_3, R_4, R_5, R_6, R_9$, n, y, x und z die obige Bedeutung haben,

umsetzt und anschließend für den Fall, daß in der Verbindung der allgemeinen Formel (I)

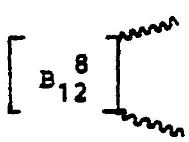 für 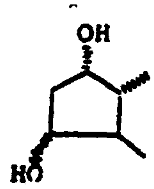 steht,

mit einer Säure gemäß dem Reaktionsschema I die eingeführten Schutzgruppen, gegebenenfalls unter Veresterung, wieder abspaltet und so Verbindungen mit der allgemeinen Formel (Ia) erhält,

oder gemäß dem Reaktionsschema II für den Fall, daß in der Verbindung der allgemeinen Formel (I)

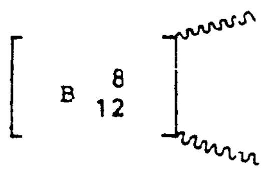 für 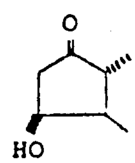 steht,

(x) zum Keton der allgemeinen Formel (XI)

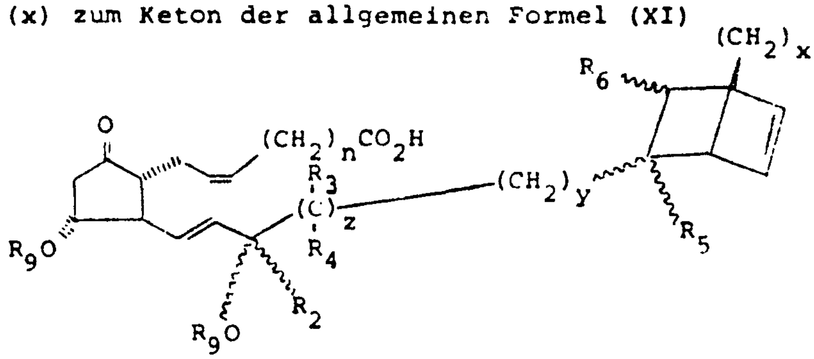

(XI)

in welcher

$R_2, R_3, R_4, R_5, R_6, R_9, n, x, y$ und $z$ die obige Bedeutung haben

oxidiert und anschließend die Schutzgruppen, gegebenenfalls unter Veresterung abspaltet und so Verbindungen der allgemeinen Formel (Ib) erhält,

oder gemäß dem Reaktionsschema II für den Fall, daß in der Verbindung der allgemeinen Formel (I)

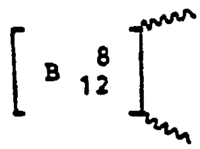 für 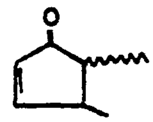 steht,

die Verbindung (Ib) oder (XI) mit einer Säure umsetzt und so die Verbindung der allgemeinen Formel (Ic) erhält,

oder gemäß dem Reaktionsschema II für den Fall, daß in der Verbindung der allgemeinen Formel (I)

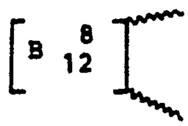 für 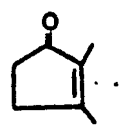 steht,

die Verbindung (Ib) mit einer Base umsetzt und so die Verbindung der allgemeinen Formel (Id) erhält.

4. Arzneimittel enthaltend mindestens ein Bicycloalkenylprostaglandin gemäß Anspruch 1.

5. Verfahren zur Herstellung von Arzneimitteln, dadurch gekennzeichnet, daß man Bicycloalkenyl-prostaglandine gemäß Anspruch 1, gegebenenfalls unter Zugabe von Hilfs- und Trägerstoffen in eine geeignete Applikationsform überführt.

6. Verwendung von Bicycloalkenyl-prostaglandinen gemäß Anspruch 1 bei der Bekämpfung von Erkrankungen.

7. Verwendung von Bicycloalkenyl-prostaglandinen gemäß Anspruch 1 bei der Bekämpfung von hormonal beeinflußten Erkrankungen.

8. Verfahren zur Behandlung von hormonal beeinflußten Erkrankungen, dadurch gekennzeichnet, daß man Bicycloalkenyl-prostaglandine gemäß Anspruch 1 Warmblütern im Bedarfsfall appliziert.

Europäisches Patentamt

# EUROPÄISCHER RECHERCHENBERICHT

Nummer der Anmeldung
EP 78 10 1396

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | CHEMICAL ABSTRACTS, Vol. 84, 1976, Columbus, Ohio, USA, HAYASHI MASAKI et al. "16-Norbornyl-17,18,19,20-tetranorprostaglandins", Seite 421, Nr. 164266b & JP - A - 75 151843 (ONO) -- | |
| A | FR - A - 2 205 338 (PFIZER) ---- | |

KLASSIFIKATION DER ANMELDUNG (Int.Cl.²)
C 07 C 177/00

RECHERCHIERTE SACHGEBIETE (Int. Cl.²)
C 07 C 177/00

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 15-02-1979 | BERTE |